# FASCICULE DE BREVET EUROPEEN

(11) **EP 0 551 048 B1**
(45) Date de publication et mention de la délivrance du brevet: **07.08.2002**
(21) Numéro de dépôt: 92420461.3
(22) Date de dépôt: 16.12.1992
(51) Int. Cl.: C07D 233/86, C07D 233/84, A01N 43/50, C07C 243/10, C07C 333/00, C07D 401/12, C07D 409/06, C07D 405/06, C07D 401/04

(54) **Dérivés de 2-imidazoline-5-one et 2-imidazoline-5-thiones fongicides**
2-Imidazolin-5-One und 2-Imidazolin-5-Thionederivate als Fungizide
2-imidazoline-5-one and 2-imidazoline-5-thiones derivatives as fungizides

(30) Priorité: 20.12.1991 FR 9116200
(43) Date de publication de la demande: 14.07.1993
(73) Titulaire: AVENTIS CROPSCIENCE S.A., 69009 Lyon (FR)
(72) Inventeur: Lacroix, Guy, F-69009 Lyon (FR); Peignier, Raymond, F-69300 Caluire (FR); Pepin, Régis, F-69140 Rillieux la Pape (FR)
(74) Mandataire: Bentham, Stephen

(56) Documents cités:
- EP-A- 0 121 054
- EP-A- 0 494 819
- WO-A-88/07040
- WO-A-90/12791
- WO-A-93/24467
- FR-A- 2 329 276
- "Dictionary of Organic Compounds", 5 édition, vol. 2, page 2058, Chapman and Hall, New York, US
- "Dictionary of Organic Compounds", 5 édition, vol. 2, page 2148, Chapman and Hall, New York, US
- "Dictionary of Organic Compounds", 5 édition, vol. 3, page 2676, Chapman and Hall, New York, US
- "Dictionary of Organic Compounds", 5 édition, vol. 4, pages 3723-3724, Chapman and Hall, New York, US
- "Dictionary of Organic Compounds", 5 édition, vol. 4, page 3890, Chapman and Hall, New York, US
- Dr. O.-A. NEUMÜLLER: "Römpps Chemie-Lexikon", pages 1805-1806, Franckh'sche Verlagshandlung, Stuttgart, DE
- CHEMICAL ABSTRACTS, vol. 113, no. 5, 30 juillet 1990, page 390, colonne 2, abrégé no. 46760c, Columbus, Ohio, US; M. SABIO et al.: "An ab initio study of amino-sulfur compounds", & THEOCHEM 1990, 65(3-4), 335-57
- D3, D4, D5: Listes de composés divulgués dans Chemical Abstracts
- YAMAMOTO J. CHEM. SOC. PERKIN TRANS. I vol. 11, 1990, pages 3003 - 3009
- Beilstein: composés avec BRN=5949625 et BRN=5930770
- MOHINDRA TETRAHEDRON vol. 51, no. 9, 1995, pages 2709 - 2718

## Description

La présente invention concerne de nouveaux composés imidazoline-ones ou imidazoline-thiones à usage phytosanitaire. Elle concerne également les procédés de préparation desdits composés et les produits éventuellement utilisables à titre d'intermédiaires dans les procédés de préparation. Elle concerne ensuite l'utilisation à titre de fongicides de ces composés, les compositions fongicides à base de ces composés et les procédés pour lutter contre les maladies fongiques des cultures utilisant ces composés.

On connaît par la demande EP 494819 (qui appartient à l'état de la technique défini par l'article 54(3) CBE) des 4,4-diméthyl-1-phényl-2-alkylthio-2-imidazoline-5-ones substituées, à action pharmaceutique.

On connaît également par la demande EP 121054 des dérivés sulfénylés d'hydantoïnes à action biocide.

Un but de la présente invention est de proposer des composés présentant des propriétés améliorées dans le traitement des maladies fongiques.

Un autre but de la présente invention est de proposer des composés présentant un spectre d'utilisation également amélioré dans le domaine des maladies fongiques.

Il a maintenant été trouvé que ces buts pouvaient être atteints grâce aux produits de l'invention, qui sont des dérivés de 2-imidazoline-5-ones ou de 2-imidazoline-5-thiones de formule générale (I) 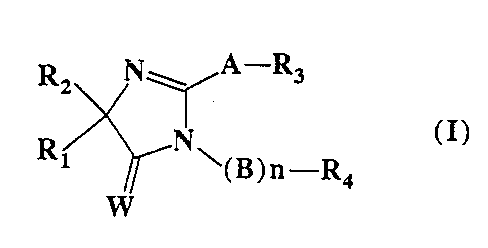 dans laquelle :
- W est l'atome de soufre ou d'oxygène ou le groupe S=O.
- A représente O ou S
- n =0 ou 1
- B représente N(R5) ou O ou S ou C(R5)(R6) ou SO2 ou C=O.

- R1 et R2, identiques ou différents représentent :
   - H, à condition qu'un des 2 groupes soit différent de H, ou
   - un radical alkyl ou haloalkyl de 1 à 6 atomes de carbone ou
   - un radical alkoxyalkyl, alkylthioalkyl, alkylsulfonylalkyl, mono alkylaminoalkyl, alcènyl ou alcynyl de 2 à 6 atomes de carbone ou
   - un radical dialkylaminoalkyl ou cycloalkyl de 3 à 7 atomes de carbone ou
   - un radical aryl, comprenant phényl, naphtyl, thiényl, furyl, pyridyl, benzothiényl, benzofuryl, quinolinyl, isoquinolinyl, ou méthylène dioxyphényl, éventuellement substitué par 1 à 3 groupements choisis parmi R7 ou
   - un radical arylalkyl, aryloxyalkyl, arylthioalkyl ou arylsulfonylalkyl, les termes aryl et alkyl ayant les définitions données ci-dessus ou
   - R1 et R2 peuvent former, avec le carbone auquel ils sont liés sur le cycle, un carbocycle ou un hétérocycle ayant de 5 à 7 atomes, ces cycles pouvant être fusionnés avec un phényl, éventuellement substitué par 1 à 3 groupes choisis parmi R7;
- R3 représente :
   - un groupe alkyl de 1 à 6 atomes de carbone ou
   - un groupe alkoxyalkyl, alkylthioalkyl, alkylsulfonylalkyl, haloalkyl, cynoalkyl, thiocyanatoalkyl, oxoalkyl, alcényl ou alcynyl, de 2 à 6 atomes de carbone ou
   - un groupe dialkylaminoalkyl, alkoxycarbonylalkyl, ou N-alkylcarbamoylalkyl de 3 à 6 atomes de carbone ou
   - un groupe N,N-dialkylcarbamoylalkyl de 4 à 8 atomes ou
   - un groupe arylalkyl, la partie alkyl étant un radical de 1 à 6 atomes de carbone et la partie aryl est phényl, naphtyl, thiényl, furyl, pyridyl, éventuellement substitué par 1 à 3 groupements choisis parmi R7;
- R4 représente :
   - l'atome d'hydrogène quand n est égal à 1 ou
   - un groupe alkyl de 1 à 6 atomes de carbone ou
   - un groupe alkoxyalkyl, alkylthioalkyl, haloalkyl, cyanoalkyl, thiocyanatoalkyl, alcényl ou alcynyl de 2 à 6 atomes de carbone ou
   - un groupe dialkylaminoalkyl, alkoxycarbonylalkyl, ou N-alkylcarbamoylalkyl de 3 à 6 atomes de carbone ou
   - un groupe N,N-dialkylcarbamoylalkyl de 4 à 8 atomes de carbone ou
   - un radical aryl, comprenant phényl, naphtyl, thiényl, furyl, pyridyl, pyrimidyl, pyridazinyl, pyrazinyl, benzothiényl, benzofuryl, quinolinyl, isoquinolinyl, ou méthylène dioxyphényl, éventuellement substitué par 1 à 3 groupements choisis parmi R7 ou
   - un radical arylalkyl, aryloxyalkyl, arylthioalkyl ou arylsulfonylalkyl les termes aryl et alkyl ayant les définitions données ci-dessus ou
   - un groupe amino disubtitué par 2 groupes identiques ou différents choisis parmi:
      - un radical alkyl de 1 à 6 atomes de carbone
      - un radical alkoxyalkyl, alcényl, ou alcynyl de 3 à 6 atomes de carbone
      - un radical cycloalkyl de 3 à 7 atomes de carbone
      - un radical arylalkyl, tel que défini ci-dessus, phényl ou naphtyl, éventuellement substitué par 1 à 3 groupements choisis parmi R7 ou
      - un radical thiénylméthyl ou furfuryl
   - un groupe pyrrolidino, pipéridino, morpholino ou pipérazino éventuellement substitué par alkyl de 1 à 3 atomes de carbone;
- R5 représente :
   - H, sauf quand R4 est H, ou
   - un radical alkyl, haloalkyl, alkylsulfonyl, haloalkylsulfonyl de 1 à 6 atomes de carbone ou
   - un radical alkoxyalkyl, alkylthioakyl, acyl, alcényl, alcynyl, haloacyl, alkoxycarbonyl, haloalkoxycarbonyl, alkoxyalkylsulfonyl, cyanoalkylsulfonyl de 2 à 6 atomes de carbone ou
   - un radical alkoxyalkoxycarbonyl, alkylthioalkoxycarbonyl, cyanoalkoxycarbonyl de 3 à 6 atomes de carbone ou
   - le radical formyl ou
   - un radical cycloalkyl, alkoxyacyl, alkylthioacyl, cyanoacyl, alcénylcarbonyl, alcynylcarbonyl de 3 à 6 atomes de carbone ou
   - un radical cycloalkylcarbonyl de 4 à 8 atomes de carbone ou
   - un radical phényl; arylalkylcarbonyl, notamment phénylacétyl et phényl propionyl;arylcarbonyl,notamment benzoyl, éventuellement substitué par 1 à 3 groupes parmi R7; thiénylcarbonyl; furylcarbonyl; pyridylcarbonyl; benzyloxycarbonyl; furfuryloxycarbonyl; tetrahydrofurfuryloxycarbonyl; thiénylméthoxycarbonyl; pyridylméthoxycarbonyl; phénoxycarbonyl ou phénylthiolcarbonyl, le phényl étant lui-même éventuellement sustitué par 1 à 3 groupement parmi R7; alkylthiolcarbonyl; haloalkylthiolcarbonyl; alkoxyalkylthiolcarbonyl; cyanoalkylthiolcarbonyl; benzylthiolcarbonyl; furfurylthiolcarbonyl; tétrahydrofurfurylthiolcarbonyl; thiénylméthylthiolcarbonyl; pyridylméthylthiolcarbonyl; ou arylsulfonyl ou
   - un radical carbamoyl éventuellement mono ou disubstitué par
      - un groupe alkyl ou haloalkyl de 1 à 6 atomes de carbone ou
      - un groupe cycloalkyl, alcényl ou alcynyl de 3 à 6 atomes de carbone ou
      - un groupe alkoxyalkyl, alkylthioalkyl ou cyanoalkyl de 2 à 6 atomes de carbone ou
      - un phényl éventuellement sustitué par 1 à 3 groupement R7;
   - un groupement sulfamoyl éventuellement mono ou disubstitué par
      - un groupe alkyl ou haloalkyl de 1 à 6 atomes de carbone ou
      - un groupe cycloalkyl, alcényl ou alcynyl de 3 à 6 atomes de carbone ou
      - un groupe alkoxyalkyl, alkylthioalkyl ou cynoalkyl de 2 à 6 atomes de carbone ou
      - un phényl éventuellement sustitué par 1 à 3 groupement R7;
   - un groupe alkylthioalkylsulfonyl de 3 à 8 atomes de carbone ou cycloalkylsulfonyl de 3 à 7 atomes de carbone;
- R6 représente :
   - l'atome d'hydrogène ou
   - le groupe cyano ou
   - un groupe alkyl de 1 à 6 atomes de carbone ou cycloalkyl de 3 à 7 atomes de carbone ou
   - un groupe acyl ou alkoxycarbonyl de 2 à 6 atomes de carbone ou
   - le groupe benzoyl éventuellement substitué par un 1 à 3 groupes R7;
- R7 représente :
   - un atome d'halogène ou
   - un radical alkyl, haloalkyl, alkoxy, haloalkoxy, alkylthio, haloalkylthio ou alkylsulfonyl de 1 à 6 atomes de carbone ou
   - un radical cycloalkyl, halocycloalkyl, alcényloxy, alcynyloxy, alcénylthio, alcynylthio de 3 à 6 atomes de carbone ou
   - le groupe nitro ou cyano ou
   - un radical amino éventuellement mono ou disubstitué par un radical alkyl ou acyl de 1 à 6 atomes de carbone ou alkoxycarbonyl de 2 à 6 atomes de carbone
   - un radical phényl, phénoxy ou pyridyloxy, ces radicaux étant éventuellement substitués;
   et leurs formes salifiées,
   à l'exclusion des composés pour lesquels lorsque R1 et R2 sont simultanément phényle ou simultanément méthyle, B est C(R5)(R6), A est le soufre, et W est O ou S;
   à l'exclusion des 3 composés avec R1 = hydrogène, n = 1, B= NR5, avec R5 et R4 = méthyle, AR3 = SCH3, W=O et R2 étant soit un hydrogène, un méthyle ou un phényle;
   à l'exclusion du composé dans lequel R1 est un phényl, R2, R3 et R4 sont méthyl, n est égal à zéro, et A et W sont chacun l'atome de soufre;
   à l'exclusion également des composés de formule (I) pour lesquels:
   - n = 0; et
   - R1 et R2 représentent simultanément un méthyle; et
   - A est un atome de soufre; et
   - R4 est un phényle substitué par 2 groupements:
      - l'un choisi parmi les groupes cyano, nitro, ou un atome d'halogène;
   - l'autre choisi parmi un groupe trifluorométhyle ou un atome d'halogéne; et à l'exclusion des composés suivants:
   - la 3-[(4-chlorophenyl)sulfonyl]-3,5-dihydro-2-methoxy-5-methyl-5(1-methylethyl)-4H-imidazol-4-one;
   - la 2-(éthoxycarbonylméthylthio)-3-acétyl-5,5-diphenyl-3,5-dihydro-4*H*-imidazol-4-one
   - la 2-butoxy-5-ethyl-3,5-dihydro-3-methyl-5-phenyl-4H-imidazol-4-one;
   - la 2-methoxy-5-methyl-3,5-dihydro-3-(1-phenylethyl)-4H-imidazol-4-one;
   - la 2-methylthio-5,5-dimethyl-3,5-dihydro-3-phenyl-4H-imidazol-4-one;
   - la 2-(methylthio)-3,5,5-triphenyl-3,5-dihydro-4*H*-imidazol-4-one;
   - la 3-methyl-2-(methylthio)-1,3-diazaspiro[4,5]dec-1-ene-4-one;
   - la 3-methyl-2-(methylthio)-1,3-diazaspiro[4,5]dec-1-ene-4-thione.

Certains composés particuliers de la formule I, de formule Ia sont connus . 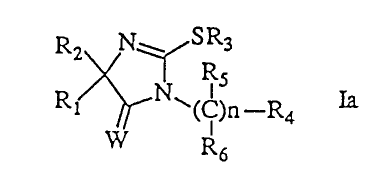 dans laquelle W,R1 à R6 et n ont la même signification que dans la formule I.

Désignés comme dérivés S-alkylés de la 5,5-diphényl-2-thiohydantoïne et de la 5,5-diphényl dithiohydantoïne, ils ont notamment été étudiés pour leurs propriétés pharmacologiques:
a) Zejc, A.; Dissertationes Pharmaceuticae et pharmacologicae. Warschau, 20 (5), 507-524 et 525-537 (1968)
b) Lucka-Sobstel, B.; Zejc, A.; Dissertationes Pharmaceuticae et pharmacologicae, 22 (1), 13-19 (1970)
c) Fetter, J.; Harsanyi, K.; Nyitrai, J.; Lempert, K.; Acta Chemica (Budapest), 78 (3), 325-333 (1973)

Aucune activité fongicide agricole n'a été décrite pour ces composés.

D'autres composés particuliers de formule I, ont été décrits par Böhme, Martin et Strahl dans Archiv der Pharmazie 313 10-15 (1980) (réf d). Il s'agit des 3 composés suivants :

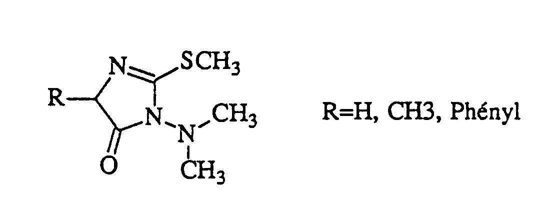

Ces 3 composés sont donc exclus des composés selon l'invention qui ont la formule Ib : 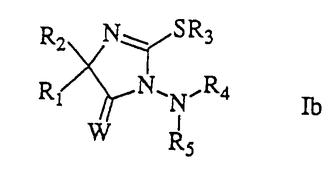 dans laquelle W, et R1 à R5 ont la même signification que dans la formule I.

Les composés de formule Ia peuvent être préparés selon les procédés en soi connus décrits dans les références citées précédemment et dans les références suivantes:
e) Biltz, H.; Chemische Berichte 42, 1792-1801 (1909)
f) Chattelain, M. et Cabrier, P.; Bulletin de la Société Chimique de France 14 (1947), 639-642
g) Carrington C. H.; Warring, W. S.; Journal of Chemical Society (1950) 354-365
h) Lampert, K.; Breuer, J.; Lemper-Streter, M.; Chemische Berichte 92, 235-239 (1959)
i) Shalaby, A.; Daboun, H.A.; Journal für Praktische Chemie; 313 (6), 1031-1038 (1971)
j) Simig, G.; Lemper, K.; Tamas, J.; Tetrahedron 29 (22), 3571-3578 (1973)
k) Schmidt, U.; Heimgartner, H.; Schmidt, H.; Helvetica Chemica Acta 62 (1979), 160-170
l) Muraoka, M.; Journal of Chemical Society Perkin Transactions I, (1990), 3003-3007
ou selon un des procédés A, B, C ou D décrits ci-dessous.

Les composés de formule Ib peuvent être obtenus selon le procédé décrit par Böhme, Martin et Strahl dans Archiv der Pharmazie 313 10-15 (1980) (Référence d) ou selon un des procédés décrits ci-dessous.

### Procédé A: Procédé de préparation des composés de formule (I).

La préparation des composés de formule (I) par S-alkylation des 2-thiohydantoïnes (II) se fait selon le schéma réactionnel : 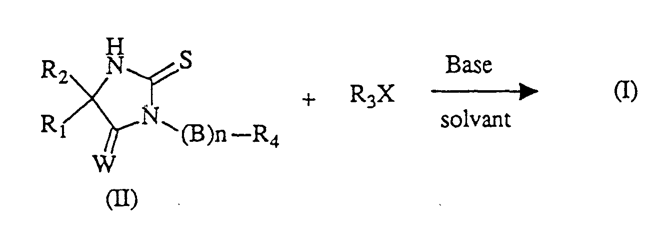 dans lequel X représente l'atome de chlore, de brome ou d'iode ou le groupe sulfate, ou un groupe alkylsulfonyloxy ou arylsulfonyloxy, alkyl et aryl étant comme définis plus haut pour R1 et R2. Comme base on peut utiliser un alcoolate, par exemple du tertiobutylate de potassium, un hydroxyde alcalin ou alcalino-terreux, un carbonate alcalin ou une amine tertiaire. Comme solvant on peut utiliser les éthers, les éthers cycliques, les esters d'alkyls, l'acétonitrile, les alcools de 1 à 3 atomes de carbone, les solvants aromatiques, par exemple le tétrahydrofurane à température comprise entre -5°C et +80°C.

Ce procédé convient pour les composés dans lesquels W représente l'atome de soufre ou d'oxygène.

Les 2-thiohydantoïnes de formule (II) peuvent être obtenues selon les procédés décrits dans la littérature comme par exemple dans les références suivantes:
e) Biltz, H.; Chemische Berichte 42, 1792-1801 (1909)
n) Eberly and Dains; Journal of American Chemical Society, 58 (1936), 2544-2547
o) Carrington C. H.; Journal of Chemical Society (1947) 681-686
g) Carrington C. H.; Warring, W. S.; Journal of Chemical Society (1950) 354-365
h) Lampert, K.; Breuer, J.; Lemper-Streter, M.; Chemische Berichte 92, 235-239 (1959)
i) Koltai, E.; Nyitrai, J.; Lempert, K.; Burics, L.; Chemische Berichte 104, 290-300 (1971)
ou bien selon un des procédés E ou F décrits ci-dessous et faisant partie de l'invention.

### Procédé B: Préparation des composés Ic..

La préparation des 2-méthylthio-2-imidazoline-5-ones de formule (Ic) par cyclisation des iminodithiocarbonates de formule (V) s'effectue selon le schéma global : 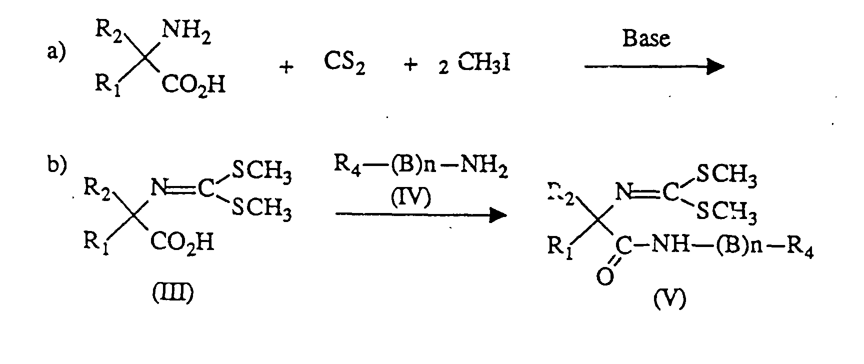 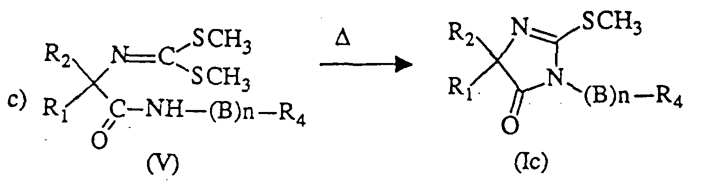
a) Les iminodithiocarbonates (III) peuvent être préparés en opérant selon les conditions décrites dans la littérature pour des composés analogues:
   - C. Alvarez Ibarra et al.; Tatrahedron Letters, 26 (2), 243-246 (1985)
   - E. Melendez et al.; Synthesis 1981, 961
      selon : 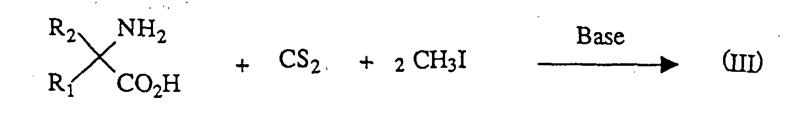
b) Les composés de formule (V) sont obtenus par condensation des composés de formule (III) avec des amines ou des hydrazines de formule (IV). Pour effectuer la condensation, l'acide (III) doit être activé sous forme de chlorure d'acide, de dicyclohexylisourée à l'aide de dicyclohexylcarbodiimide ou d'imidazolide à l'aide de carbonyldiimidazole. La condensation est effectuée dans les conditions habituelles pour ce genre de réaction.
c) La cyclisation des composés (V) est réalisée par simple chauffage dans un solvant aromatique à reflux. Comme solvant on peut notamment utiliser le xylène, le chlorobenzène ou le dichlorobenzène.

### Procédés C: Dérivatisation des composés (Ib') et (Id').

### Procédé C1 : Préparation des composés Ib par N dérivatisation des composé Ib'.

Les composés de formule (Ib') (composés (Ib) dans lesquels R5 est l'atome d'hydrogène) peuvent être alkylés, acylés, alcoxycarbonylés, carbamoylés ou sulfamoylés selon le schéma général suivant:

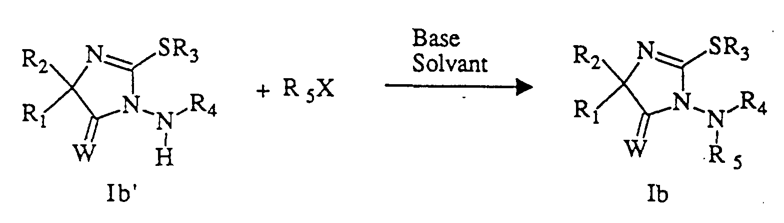

R5 représente ici un groupe alkyl, alcoxycarbonyl, acyl, arylcarbonyl, alkylsulfonyl, arylsulfonyl, carbamoyl ou sulfamoyl,tels que définis ci-dessus.

X représente, un halogène, le groupe sulfate ou un phénoxy éventuellement substitué, ou un groupe alkylsulfonyloxy ou arylsulfonyloxy, ou un groupe R5O, quand R5 est acyle.

Comme base on peut utiliser les hydrures alcalins, les alcoolates ou une amine tertiaire. La réaction peut être pratiquée à température comprise entre -30°C et +50°C. Comme solvant on peu utiliser par exemple les éthers, les éthers cycliques, le diméthylformamide, le diméthylsulfoxyde, les solvants aromatiques.

La carbamoylation des composés (Ib') peut être effectuée par réaction avec les isocyanates ou les isothiocyanates selon le schéma:

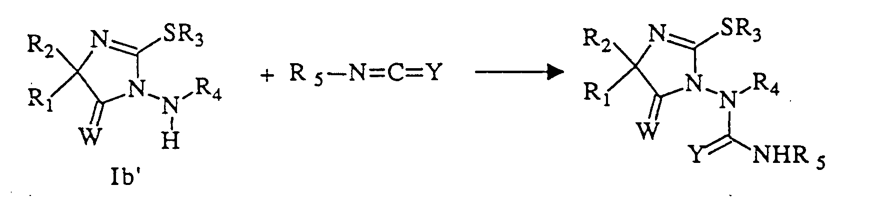

La réaction est pratiquée dans les mêmes conditions que celles décrites ci-dessus, la base pouvant cependant être utilisée en quantité catalytique.

### Procédé C2 : Préparation des composés Id.

Les composés Id' (composés Id dans lesquels R2 est l'atome d'hydrogène) peuvent être alkylés en position 4 selon le schéma :

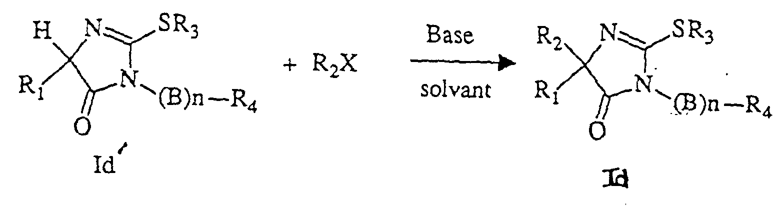

X représente un atome de chlore, brome ou iode. Comme base on peut utiliser un alcoolate, un hydrure métallique ou un amidure. La réaction peut être pratiquée à température comprise entre -30°C et +80°C. Comme solvant on peut utiliser les éthers, les éthers cycliques, le diméthylformamide, le diméthylsulfoxyde, les solvants aromatiques.

### Procédé D: Préparation des dérivés S-oxydés des 2-imidazoline-5-thiones.

Les composés de formule (I) dans lesquels W représente le groupe S=O sont obtenus par oxydation des 2-imidazoline-5-thiones selon le schéma:

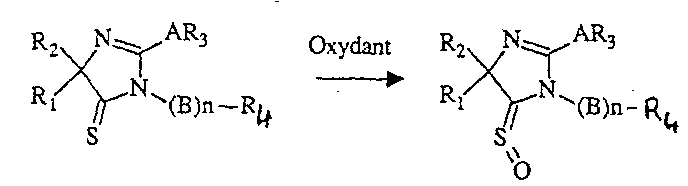

Comme oxydant on peut utiliser les peroxydes, notamment les peracides. L'oxydant doit être mis en oeuvre en quantité stoechiométrique. L'oxydation est effectuée dans le chloroforme ou dans le chlorure de méthylène à température comprise entre -20°C et +20°C.

### Procédé E: Préparation des dithiohydantoïnes de formule (VI).

Les dithiohydantoïnes de formule (VI) peuvent être obtenues par piégeage des anions en alpha des isothiocyanates par des isothiocyanates non anionisable selon le schéma:

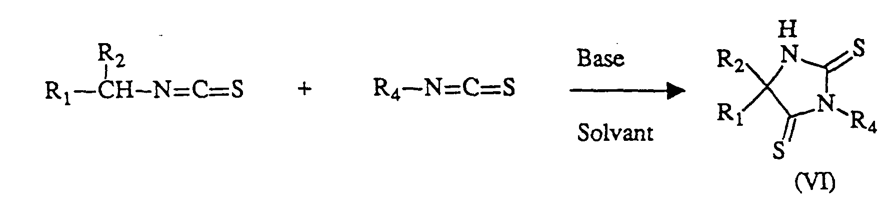

Au moins un des groupes R1 ou R2 doit être électro-attracteur ( Aryl, aryl substitué, alcoxycarbonyl...). L'isothiocyanate R4-NCS ne doit pas être anionisable; les arylisothiocyanates sont particulièrement utilisables dans cette réaction.

Comme base on peut utiliser le tertiobutylate de potassium, le bis(triméthylsilyl) amidure de lithium ou de sodium, les hydrures alcalins. Comme solvant on peut utiliser les éthers, les éthers cycliques. La réaction est pratiquée à température inférieure à -60°C. L'anion doit être piégé au fur et à mesure de sa formation. Pour celà on coule le mélange des 2 isothiocyanates sur la base en solution à température inférieure à -60°C.

### Procédé F: Préparation des 2-thiohydantoïnes de formule (VII).

La préparation des 2-thiohydantoïnes (VII) à partir des isothiocyanates dérivés des amino acides (VIII) se fait selon la réaction :

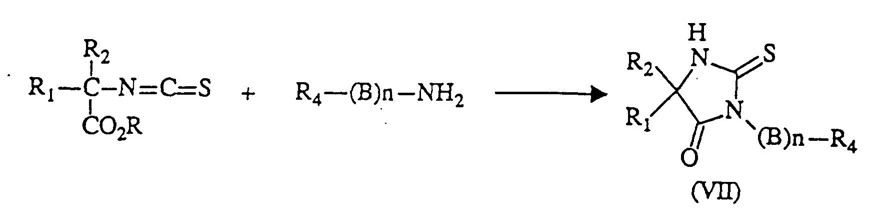

La cyclisation peut être réalisée de deux façons:
- thermiquement: dans ce cas on chauffe le mélange des réactifs à température comprise entre 110°C et 180°C dans un solvant aromatique tel que le toluène, le xylène, les chlorobenzènes.
- en milieu basique: on procède en présence d'un équivalent d'une base telle qu'un alcoolate alcalin, un hydroxyde alcalin ou une amine tertiaire. Dans ces conditions la cyclisation se produit à température comprise entre -10 et +80°C. Comme solvant on peut utiliser les éthers, les éthers cycliques, les alcools, les esters, le DMF, le DMSO....

Les isothiocyanates peuvent être préparés selon un des procédés cités dans Sulfur Reports Volume 8 (5) pages 327-375 (1989).

### Procédé G: Préparation des composés de formule I en une étape.

Lors de la cyclisation des 2-thiohydantoïnes selon le procédé F, si l'on opère en milieu basique, en fin de réaction la thiohydantoïne se trouve sous forme de thiolate que l'on peut faire réagir directement avec un halogénure ou un sulfate d'alkyle R3X ou avec R3X dans lequel X est un alkylsulfonyloxy ou un arylsulfonyloxy pour former (I). On enchaine ainsi les procédés A et F selon le schéma:

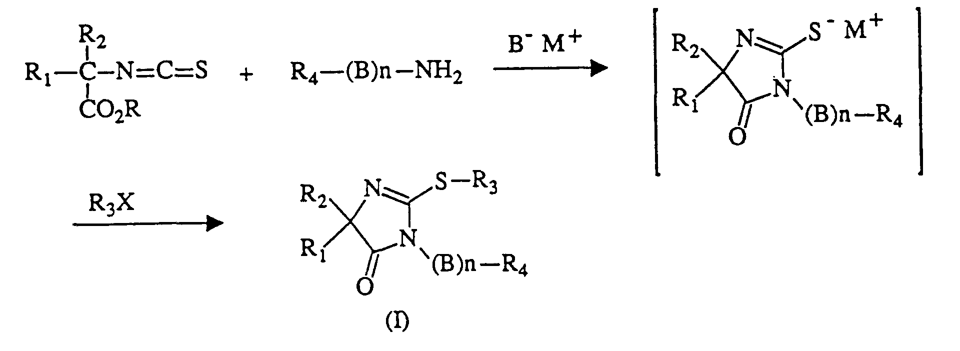

### Procédé H: Préparation des composés de formule Ie, dans laquelle (B)n est un atome de soufre:

Ces composés peuvent être obtenus par réaction d'un chlorure de sulfuryle R₄SCl avec une imidazolinone de formule IX selon le schéma:

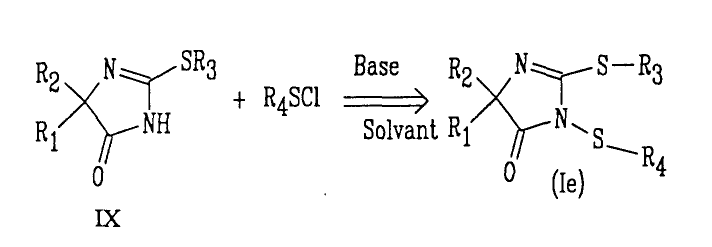

La réaction est effectuée à température comprise entre -20°C et +30°C, en présence d'un équivalent molaire d'une base. Comme base, on peut utiliser les hydrures alcalins, les alcoolates alcalins ou les amines tertiaires. Comme solvant on peut utiliser des solvant polaires par exemple les éthers, les éthers cycliques, le diméthylformamide, le diméthylsulfoxyde, ou des solvants aromatiques. Les imidazolinones (IX) peuvent être préparées par des procédés analogues au procédé A.

Les composés préférés pour leur meilleure activité fongicide et / ou pour leur facilité de synthèse sont:
1) les composés de formule Ib,
2) les composés de formule I, en particulier Ib, dans lesquels R5 est l'atome d'hydrogène,
3) les composés dans lesquels R1 et R2 sont différents de H,
4) les composés dans lesquels R2 représente un groupe alkyl de 1 à 3 atomes de carbone,
5) les composés dans lesquels R1 représente le noyau phényl, éventuellement substitué par R7,
6) les composés dans lesquels R3 représente un groupe alkyl de 1 à 3 atomes de carbone,
7) les composés dans lesquels R4 représente le noyau phényl, éventuellement substitué par R7,
8) les composés dans lesquels R3 représente le groupe méthyl.

Les composés qui répondent aux formules IIa, III, V et VIIa dans lesquelles W, R1, R2, R4 et R5, n et B sont tels que définis dans la formule I, R4 étant autre que l'atome d'hydrogène,
à l'exclusion des composés selon formule IIa avec:
W = S,
R¹ = méthyl, R² = phényl, R⁵ = hydrogène et R⁴ = methyl ou phenyl;
W=S,
R¹ = ethyl, R² = phenyl, R⁴ = methyl et R⁵ hydrogène;
W=S,
R¹ = methyl, R² = p-tolyl, et
R⁴ = methyl, R⁵ = hydrogène ou methyl ou
R⁴ = phenyl, R⁵ = hydrogène;
W=S,
R¹ et R² = pheny R⁴ = methyl et R⁵ = H,
et à l'exclusion des composés selon la formule VIIa avec:
R₄ = methyl, R₅ = methyl, R₁ = hydrogen
et R₂ = methyl ou phenyl; sont aussi des composés selon l'invention.

Les composés préferes sont:
1) les composés dans lesquels:
   R⁵ est l'atome d'hydrogène,
   R² représente un groupe alkyl de 1 à 3 atomes de carbone,
   R₁ représente le noyau phenyl, éventuellement substitute par R⁷,
   R⁴ représente le noyau phényl, éventuellement substitué par R⁷, et
   R₇ est tel que défini ci-dessus;
2) les composés de formule VIIa;
3) les composés dans lesquels R₂ représente le groupe methyl;
4) les composés dans sequels R₁ représente le noyau phenyl.

Les exemples ci-après sont donnés à titre d'illustration des composés selon l'invention, de leurs procédés de préparation et de leurs propriétés . antifongiques.

Les structures de tous les produits ont été établies par au moins 1 des techniques spectrales suivantes: spectrométrie RMN du proton, spectrométrie RMN du carbone 13, spectrométrie Infra-Rouge et spectrométrie de masse.

Dans les tableaux ci-après, les radicaux méthyl et phényl sont respectivement représentés par Me et Ph, et Cte signifie constante physique c'est à dire soit un point de fusion (PF) soit l'indice de réfraction (nD²⁰).

### Exemple 1: Préparation du composé N° 34 selon le procédé A.

On dissout 0,9 g (3 mmoles) de 3-benzyl-5-méthyl-5-phényl 2-thiohydantoïne dans 30 ml de tétrahydrofurane anhydre. On refroidit le milieu à 0°C puis on ajoute 0,34 g (3 mmoles) de tertiobutylate de potassium. On laisse réagir 10 mn à 0°C puis on coule goutte à goutte à cette température 0,46 g (3,3 mmoles) de iodure de méthyle : on observe la précipitation de l'iodure de potassium. On laisse la température du milieu remonter à l'ambiante. On dilue le milieu avec 100 ml d'acétate d'éthyle. On lave la solution 2 fois avec 100 ml d'eau chacune. On sèche la solution sur sulfate de sodium puis traite celle-ci au charbon actif. On concentre la solution sous pression réduite : on récupère 0,6 g de 1-benzyl-4-méthyl-2-méthylthio-4-phényl-2-imidazoline-5-one (composé N° 34) sous forme d'un solide jaune pâle fondant à 68°C.

De la même manière, nous avons préparé les composés décrits ci-après:

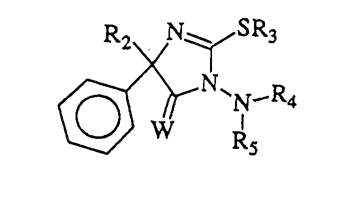

| **N°** | **R2** | **R3** | **R4** | **R5** | **W** | **Cte** |
|---|---|---|---|---|---|---|
| 1 | Me | Me | Ph | H | S | PF=127°C |
| 3 | Me | 2-Oxopropyl | Ph | H | S | PF=130°C |
| 9 | Me | Me | Ph | H | O | PF=149°C |
| 10 | Me | Me | métatolyl | H | O | PF=124°C |
| 11 | Me | Me | paratolyl | H | O | PF=150°C |
| 12 | Me | Et | Ph | H | O | PF=118°C |
| 13 | Me | Me | 4-FluoroPh | H | O | PF=144°C |
| 14 | Me | Allyl | Ph | H | O | PF=92°C |
| 15 | Me | Me | orthotolyl | H | O | PF=92°C |
| 16 | Me | Me | 3-ChloroPh | H | O | PF=120°C |
| 17 | Me | isoPropyl | Ph | H | O | PF=95°C |
| 18 | Me | Me | 4-ChloroPh | H | O | PF=149°C |
| 19 | Me | Me | tertiobutyl | H | O | PF=73°C |
| 20 | Me | Me | 2-ChloroPh | H | O | PF=134°C |
| 22 | Me | Me | Ph | Me | O | PF=124°C |
| 23 | Me | Me | Ph | Acétyl | O | PF=132°C |
| 24 | Me | Me | 4-méthoxyPh | H | O | PF=138°C |
| 25 | Me | npropyl | Ph | H | O | PF=90°C |
| 40 | Me | Me | 2-méthoxyPh | H | O | PF=110°C |
| 41 | Me | Me | Acétyl | H | O | PF=55°C |
| 43 | Me | Me | 4-NO2-Ph | H | O | PF=133°C |
| 44 | Me | Me | 2-Pyridyl | H | S | PF=114°C |
| 45 | Me | Me | 2-Pyridyl | H | O | PF=147°C |
| 46 | Me | Me | 3-Pyridyl | H | O | PF=140°C |
| 47 | Me | Me | 3-Pyridyl | H | S | PF=176°C |
| 54 | Me | Me | 2,6Me2Ph | H | S | PF=146°C |
| 73 | Me | Me | 2-Thia zolyl | Me | O | PF=116°C |
| 75 | Me | CHF2 | Ph | H | O | PF=80°C |

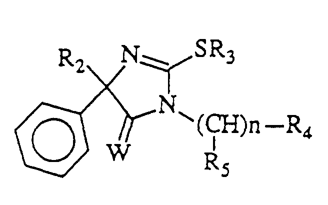

| **N°** | **R2** | **R3** | **R4** | **n** | **R5** | **W** | **Cte** |
|---|---|---|---|---|---|---|---|
| 26 | Me | Me | Ph | 0 | - | S | PF=123°C |
| 27 | Ph | Me | Ph | 0 | - | S | PF=120°C |
| 28 | Me | Me | Me | 0 | - | S | PF=85°C |
| 29 | Ph | Me | Me | 0 | - | S | PF=144°C |
| 30 | Me | Me | Ph | 0 | - | O | PF=70°C |
| 31 | Me | Me | Me | 0 | - | O | PF=58°C |
| 32 | Ph | Me | Me | 0 | - | O | PF=170°C |
| 33 | H | Me | Ph | 0 | - | O | PF=250°C |
| 34 | Me | Me | Ph | 1 | H | O | PF=68°C |
| 35 | Me | Me | 2-thiényl | 1 | H | O | PF=76°C |
| 36 | Me | Me | Me | 1 | Me | O | nD²⁰=1,553 |
| 37 | Me | Me | 2-Furyl | 1 | H | O | miel |
| 38 | Me | Me | 3-Pyridyl | 0 | - | O | miel |
| 50 | Ph | Me | Me | 0 | - | S | PF=144°C |
| 57 | Me | Me | 2-MePh | 0 | - | O | miel |

Ont également été préparées:
- la 4-(3-pyridyl)-4-méthyl-1-(N-phénylamino)-2-méthylthio-2-imidazoline-5-one(composé 51: PF 156°C);
- la 4-phényl-4-méthyl-1-(benzyloxy)-2-méthylthio-2-imidazoline-5-one(composé 56: miel).

### Exemple 2: Préparation du composé N°7 selon le procédé B.

### a) N-[bis(méthylthio)méthylène]-2-phényl glycine (composé (III) avec R1=phényl et R2=H):

On dissout à +5°C 100 g (0,66 mole) de phénylglycine dans 335 g de potasse aqueuse à 22 % (1,3 moles). On ajoute 55,3 g de sulfure de carbone tout en agitant fortement le milieu: un précipité apparait et le milieu se colore en orange. On laisse réagir 3 h à température ambiante puis coule 103 g (0,73 mole) d'iodure de méthyle tout en maintenant la température du milieu en dessous de 30°C. On laisse réagir 0,5 h puis ajoute 74 g (0,66 mole) d'une solution à 50% de potasse. On laisse réagir 0,5 h puis coule à nouveau 103 g d'iodure de méthyle et laisse réagir 1h. On dilue le milieu avec 300 ml d'eau. On acidifie à pH=4 avec de l'acide chlorhydrique 1N. On extrait le produit avec 500 ml d'acétate d'éthyle. On sèche la solution sur sulfate de magnésium puis cencentre sous pression réduite. On récupère 49,5 g de N-[bis(méthylthio)méthylène]-2-phényl glycine ( rendement=31%) sous forme d'un solide jaune fondant à 112°C.

### b) [N-(bis(méthylthio)méthylène)-2-phényl glycyl] métachlorophényl hydrazide (composé V avec R1=phényl, R2=H, R4=métachlorophényl, n=1, B=NH):

A une solution de N-[bis(méthylthio)méthylène]-2-phényl glycine 2,95 g (16,4 mmoles) dans le chlorure de méthylène (40 ml) on ajoute 3,38:g (16,4 mmoles) de dicyclohexylcarbodiimide, puis on laisse réagir 0,5 h à température ambiante. On ajoute 2,34 g (16,4 mmoles) de métachlorophénylhydrazine. On chauffe 0,5 h à 30°C. On filtre l'insoluble. On lave le filtrat avec 2 fois 30 ml d'eau chacune. On concentre la solution : on obtient un miel que l'on purifie par chromatographie sur colonne de silice. Après purification on récupère 2,5 g de [N-(bis(méthylthio)méthylène)-2-phényl glycyl] métachlorophényl hydrazide sous forme d'une poudre rosée fondant à 146°C.

### c) 1-Métachlorophényl-2-méthylthio-4-phényl-2-imidazoline-5-one (composé N°7)

On dissout 1,92 g (5 mmoles) de [N-(bis(méthylthio)méthylène)-2-phényl glycyl] métachlorophényl hydrazide dans 30 ml de xylène. On chauffe le milieu réactionnel 4 h à reflux. On concentre le milieu sous pression réduite. On triture le miel résultant avec 10 ml d'éther : le produit cristallise. On filtre le précipité, et sèche le produit en dessicateur sous vide. On obtient ainsi, avec un rendement de 56% le composé N°7 sous forme d'une poudre jaune fondant à 196°C.

En opérant de façon similaire nous avons préparé les composés figurant dans le tableau suivant:

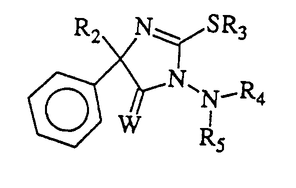

| **N°** | **R2** | **R3** | **R4** | **R5** | **W** | **Cte** |
|---|---|---|---|---|---|---|
| 4 | H | Me | 2-ChloroPh | H | O | PF=130°C |
| 5 | H | Me | Ph | H | O | PF=190°C |
| 6 | H | Me | 4-ChloroPh | H | O | PF=162°C |
| 7 | H | Me | 3-ChloroPh | H | O | PF=196°C |
| 8 | H | Me | métatolyl | H | O | PF=182°C |
| 59 | H | Me | 2,4-(cH3)2Ph | H | O | PF=64°C |
| 61 | H | Me | 2,5-(CH3)2Ph | H | O | PF=162°C |
| 63 | H | Me | 2-EtPh | H | O | PF=1266C |
| 69 | H | Me | 2,5-(Cl)2Ph | H | O | PF=144°C |
| 71 | H | Me | 3,5-(Cl)2Ph | H | O | PF=146°C |

A également été préparée la 4-phényl-1-(N-phénylamino)-2-méthylthio-2-imidazoline-5-one(composé 120).

### Exemple 3: Préparation de 4-méthyl-1-(N-méthyl-N-phénylamino)-2-méthylthio-4-phényl-2-imidazoline-5-one (composé N°22) par alkylation (méthylation) selon le procédé C1.

A une solution de 4-méthyl-1-phénylamino-2-méthylthio-4-phényl-2-imidazoline-5-one (composé N°9) (1 g; 3,2 mmoles) dans le tétrahydrofurane anhydre (30 ml), préalablement refroidie à 0°C on ajoute 0,4 g (3,5 mmoles) de tertiobutylate de potassium. On laisse réagir 0,5 h à 0°C. On ajoute ensuite 0,5 g (3,5 mmoles) d'iodure de méthyle puis on laisse réagir 0,5h à température ambiante. On verse le milieu réactionnel dans 100 ml d'eau et on extrait le produit avec 100 ml d'éther diéthylique. La solution éthérée est sèchée sur sulfate de magnésium puis concentrée. Le produit cristallise en le triturant dans 10 ml de diisopropyléther. Il est filtré puis séché sous vide. On obtient ainsi 0,73 g (Rendement:70%) du composé 22 sous forme d'une poudre jaune pâle fondant à 124°C.

### Exemple 4: Préparation de 4-méthyl-1-(N-acétyl-N-phénylamino)-2-méthylthio-4-phényl-2-imidazoline-5-one (composé N°23) par acylation (acétylation) selon le procédé C1.

A une solution de 4-méthyl-1-phénylamino-2-méthylthio-4-phényl-2-imidazoline-5-one (composé N°9) (1 g; 3,2 mmoles) dans le tétrahydrofurane anhydre (30 ml), préalablement refroidie à 0°C on ajoute 0,4 g (3,5 mmoles) de tertiobutylate de potassium. On laisse réagir 0,5 h à 0°C. On ajoute ensuite 0,25 g (3,5 mmoles) de chlorure d'acétyle puis on laisse réagir 0,5h à température ambiante. On verse le milieu réactionnel dans 100 ml d'eau et on extrait le produit avec 100 ml d'éther diéthylique. On lave la solution éthérée à l'eau jusqu'à neutralité. On sèche celle-ci sur sulfate de magnésium puis on concentre. On obtient un miel que l'on purifie par chromatographie sur colonne de silice. Le produit purifié cristallise dans le diisopropyléther. On obtient 0,25 g du composé N°23 sous forme d'une poudre blanche fondant à 132°C.

En opérant de la même façon nous avons obtenu les composés n° 39 et 42.

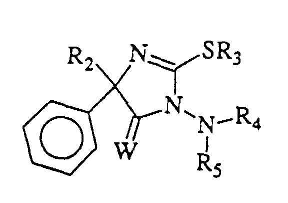

| **N°** | **R2** | **R3** | **R4** | **R5** | **W** | **Cte** |
|---|---|---|---|---|---|---|
| 23 | Me | Me | Ph | Acétyl | O | PF=132°C |
| 39 | Me | Me | Ph | Formyl | O | miel |
| 42 | Me | Me | Ph | tBuOCO | O | miel |

### Exemple 5 : Préparation de 4-éthyl-2-méthylthio-4-phényl-1-phénylamino 2-imidazoline-5-one (composé 48) selon le procédé C2.

A une solution de 1,5 g (5,05 mmoles) de 2-méthylthio-4-phényl-1-phénylamino 2-imidazoline-5-one (composé n° 5) dans 50 ml de tétrahydrofurane anhydre, on ajoute 0,55 g de tertiobutylate de potassium. On laisse réagir 30 mn à température ambiante puis ajoute 0,8 g (5,05 mmoles) de iodure d'éthyle. On laisse réagir 1 h à température ambiante. On dilue le milieu avec 150 ml d'acétate d'éthyle. On lave la solution à l'eau puis concentre sous pression réduite. On purifie le produit par chromatographie sur colonne de silice (silice Merck 60H; éluant: acétate d'éthyle 25%/heptane 75%). On obtient 0,65 g du composé n° 48 sous forme d'une poudre beige fondant à 147°C.

En opérant de la même façon nous avons obtenu le composé n° 49.

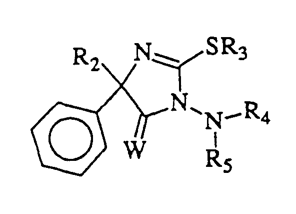

| **N°** | **R2** | **R3** | **R4** | **R5** | **W** | **Cte** |
|---|---|---|---|---|---|---|
| 48 | Et | Me | Ph | H | O | PF=147°C |
| 49 | isoPr | Me | Ph | H | O | PF=135°C |
| 60 | Me | Me | 2,4-(Me)2Ph | H | O | miel |
| 62 | Me | Me | 2,5-(Me)2Ph | H | O | PF: 160°C |
| 64 | Me | Me | 2-EtPh | H | O | miel |
| 65 | Me | Me | 2,4-(Cl)2Ph | H | O | |
| 66 | Me | Me | 1-naphtyl | H | O | PF: 174°C |
| 70 | Me | Me | 2,5-(Cl)2Ph | H | O | PF: 180°C |
| 72 | Me | Me | 3,5-(Cl)2Ph | H | O | PF: 200°C |
| 74 | CHF2 | Me | Ph | H | O | PF: 124°C |
| 79 | Me | Me | 2-CF3-Ph | H | O | PF: 91°C |

A également été préparée la 4-méthyl-2-méthylthio-4-(4-fluorophényl)-1-phénylamino 2-imidazoline-5-one(composé 68).

### Exemple 6: Préparation du composé 2 selon le procédé D.

On dissout 1,7 g (5,2 mmoles) de 4-méthyl-2-méthylthio-4-phényl-1-phénylamino-2-imidazoline-5-thione (composé N°1) dans 20 ml de chloroforme. On refroidit la solution -10°C puis on additionne en 10 mn une solution de 1,35 g (5,5 mmoles) d'acide métachloroperbenzoïque et 30 ml de chloroforme. Après la fin d'addition on laisse la température remonter à l'ambiante. On lave le milieu avec une solution aqueuse saturée de bicarbonate de sodium puis à l'eau distillée. On traite la phase organique au charbon actif puis concentre. On reprend le miel résultant avec 20 ml d'éther : celui-ci se dissoud puis un solide beige précipite. On filtre le précipité. On sèche le produit sous pression réduite. On obtient ainsi 0,4 g (rendement: 25%) du composé N°2 sous forme d'une poudre beige fondant à 150°C.

| **N°** | **R2** | **R3** | **R4** | **R5** | **W** | **Cte** |
|---|---|---|---|---|---|---|
| 2 | Me | Me | Ph | H | S=O | PF=150°C |

### Exemple 7: préparation de 3,5-diphényl-5-méthyl dithiohydantoïne selon le procédé E.

Dans un ballon tricol de 500 ml, sous atmosphère d'argon sec, on dissout 15,1 g (122 mmoles) de tertiobutylate de potassium dans 200 ml de tétrahydrofurane. On refroidit la solution à -70°C. On coule goutte à goute, en maintenant la température du milieu inférieure à -60°C, une solution de 20 g (122 mmoles) d'alphaméthylbenzylisothiocyanate, de 16,55 g (122 mmoles) de phénylisothiocyanate et de 50 ml de tétrahydrofurane. Après la fin de l'addition on maintient le milieu 0,5 h à -70°C puis on laisse revenir à température ambiante. on verse le milieu dans 500 ml d'eau. On acidifie à pH=1 par ajout d'acide chlorhydrique N. On extrait le produit avec de l'acétate d'éthyle (2 extractions avec 150 ml de solvant chacune). On sèche la solution sur sulfate de magnésium. On concente sous pression réduite. On fait cristalliser le produit dans 50 ml d'éther. On filtre le précipité. On obtient ainsi 21 (rendement: 58%) de 3,5-diphényl-5-méthyl dithiohydantoïne, poudre jaune fondant à 157°C.

### Exemple 8 : Préparation de 3,5-diphényl-5-méthyl-2-thiohydantoïne selon le procédé F.

On dissout 4,7 g (20 mmoles) de 2-isothiocyanato-2-phényl propionate d'éthyle dans 40 ml de xylène. On ajoute 2,16 g (20 mmoles) de phénylhydrazine puis chauffe 4 h à reflux. On refroidit à température ambiante, un solide beige précipite. On filtre le précipité; le lave avec 5 ml de diisopropyléther puis le sèche sous vide. On obtient ainsi 4,6 g (rendement=77%) de 3,5-diphényl-5-méthyl-2-thiohydantoïne sous forme d'une poudre beige fondant à 164°C.

### Exemple 9 : Préparation de 5-méthyl-5-phényl-3-(2-pyridylamino)-2-thiohydantoïne selon le procédé F.

On dissout 2 g (9 mmoles) de 2-isothiocyanato-2-phényl propionate de méthyle dans 30 ml de tétrahydrofurane. On ajoute une solution de 0,99 g de 2-hydrazinopyridine et de 10 ml de tétrahydrofurane : la température du milieu s'élève de 20 à 30°C et un solide précipite. On laisse réagir 0,5 h à 30°C puis on refroidit à 5°C. On ajoute alors une solution de 1 g de tertiobutylate de potassium et de 10 ml de tétrahydrofurane : coloration violette du milieu. On laisse revenir à température ambiante et laisse réagir 2 h. On verse le milieu dans 150 ml d'eau. On neutralise le milieu avec de l'acide acétique. On extrait le produit avec 150 ml d'acétate d'éthyle. On lave la solution à l'eau, la sèche sur sulfate de magnésium puis la traite au charbon actif. On concentre et fait cristalliser le produit dans 20 ml d'éther diéthylique. On filtre le précité puis le sèche sous vide. On obtient 1,6 g (rendement : 60%) de 5-méthyl-5-phényl-3-(2-pyridylamino)-2-thiohydantoïne, solide jaune pâle fondant à 80°C.

Selon ce procédé nous avons préparé les composés de formule (VII), intermédiaires des composés de formule I et numérotés à partir du numéro 1001, rassemblés dans le tableau suivant : 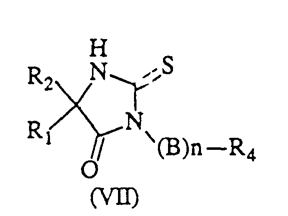 R1 = méthyl et R2 = phényl.

| **N°** | **n** | **B** | **R4** | **Rdt** | **PF** |
|---|---|---|---|---|---|
| 1001 | 1 | NH | Ph | 66% | 164°C |
| 1002 | 1 | NH | métatolyl | 62% | 174°C |
| 1003 | 1 | CH2 | Ph | 46% | 125°C |
| 1004 | 1 | NH | paratolyl | 13% | 162°C |
| 1005 | 1 | CH2 | 2-thiényl | 49,5% | 134°C |
| 1006 | 1 | NH | 4-FluoroPh | 30% | 162°C |
| 1007 | 1 | NH | orthotolyl | 38% | 162°C |
| 1008 | 0 | - | isopropyl | 60,5% | 146°C |
| 1009 | 1 | NH | 3-ChloroPh | 32% | 78°C |
| 1010 | 1 | NH | Tertiobutyl | 18% | 120°C |
| 1011 | 1 | NH | 4-ChloroPh | 24% | 196°C |
| 1012 | 1 | NH | 2-ChloroPh | 69% | 172°C |
| 1013 | 0 | - | Pipéridino | 32% | 206°C |
| 1014 | 1 | NH | 4-MéthoxyPh | 27% | 146°C |
| 1015 | 1 | NH | 2-MéthoxyPh | 29% | 214°C |
| 1016 | 1 | CH2 | 2-Furyl | 39% | 105°C |
| 1017 | 1 | NH | Acétyl | 42% | 200°C |
| 1018 | 1 | NH | 4-NO2-Ph | 41% | 234°C |
| 1019 | 1 | NH | 2-pyridyl | 60% | 80°C |
| 1020 | 1 | NH | 3-pyridyl | 17% | |

### Exemple 10 : Préparation du composé 9 selon le procédé G.

On dissout 11,1 g (50 mmoles) de 2-isothiocyanato-2-phényl propionate de méthyle dans 150 ml de tétrahydrofurane anhydre. On ajoute progressivement en 10 mn une solution de 5,4 g (50 mmoles) de phényl hydrazine et de 50 ml de tétrahydrofurane anhydre : la température du milieu s'élève jusqu'à 35°C. Après la fin d'addition on laisse réagir 0,5 h à 30°C puis on refroidit le milieu à -5°C. On ajoute à cette température une solution de 5,6 g (50 mmoles) de tertiobutylate de potassium et de 50 ml de tétrahydrofurane anhydre: le milieu se colore en violet puis un précipité se forme. On laisse réagir 0,5 h à 0°C puis ajoute 8,5 g (60 mmoles) d'iodure de méthyle. On laisse réagir 1 h à température ambiante. On dilue le milieu avec 200 ml d'acétate d'éthyle. On lave le milieu 2 fois avec 150 ml d'eau chacune. On sèche la solution sur sulfate de magnésium puis la traite au charbon actif. On concentre: on obtient un miel brun violacé que l'on fait cristalliser dans 50 ml d'éther. Le précipité est lavé puis sèché sous vide. Un deuxième jet de produit est récupéré après concentration des eaux mères et reprise du miel résiduel avec 50 ml de diisopropyléther. On obtient ainsi 12 g (rendement=77%) de 4-méthyl-2-méthylthio-4-phényl-1-phénylamino 2-imidazoline-5-one (composé 9) sous forme d'une poudre beige fondant à 149°C.

En opérant comme précédemment on a obtenu les composés suivants:

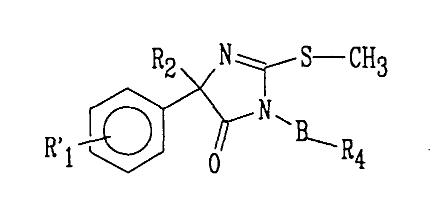

| **N°** | **R'1** | **R2 B** | | **R4** | **PF** |
|---|---|---|---|---|---|
| 58 | - | Me | NH | 2,3-(Me)2Ph | 116°C |
| 67 | - | Me | (CH2)2 | Ph | miel |
| 76 | - | Me | CH2 | 3-Pyridyl | 67 °C |
| 77 | - | Me | CH2 | 2-Pyridyl | miel |
| 78 | - | Me | N | PhCH= | 95 °C |
| 83 | 4-Me | Me | NH | Ph | 179 °C |
| 84 | - | Me | NH | 3-Me-2-Pyridyl | 148 °C |
| 85 | 4-Cl | Me | NH | Ph | 173 °C |
| 86 | 3,4-(MeO)2 | Me | NH | Ph | 165 °C |
| 87 | 3,4-(MeO)2 | Me | NH | 2-Me-Ph | 151 °C |
| 88 | 4-Me | Me | NH | 2-Me-Ph | 52 °C |
| 89 | 4-PhO | Me | NH | Ph | 146 °C |
| 90 | 4-Cl | Me | NH | 3-Me-2-Pyridyl | 133 °C |
| 91 | 4-Cl | Me | NH | 2-Pyridyl | 172 °C |
| 93 | 4-PhO | Me | NH | 2-Me-Ph | 130 °C |
| 94 | 4-F | Me | NH | 2-Me-Ph | 120 °C |
| 96 | 4-Cl | Me | NH | 2-Cl-Ph | 145 °C |
| 97 | - | (CH2)3 | NH | Ph | 158 °C |
| 99 | 4-Cl | H | NH | 4-Cl-Ph | 163 °C |
| 100 | 4-Cl | Me | NH | 4-Cl-Ph | 172 °C |
| 101 | 4-Cl | Me | NH | 4-F-Ph | 170 °C |
| 102 | 4-Cl | Me | NH | 3-Cl-Ph | 146 °C |
| 103 | 4-Cl | Me | NH | 4-Me-Ph | 178 °C |
| 104 | - | (CH2)3 | NH | 2-Cl-Ph | 168 °C |
| 107 | 4-F | Me | NH | 3-Me-Ph | 121 °C |
| 108 | - | Me | NH | 3-F-Ph | 163 °C |
| 109 | - | Me | NH | 2,5-F2-Ph | 141 °C |
| 110 | 4-Me | Me | NH | 4-Cl-Ph | 168 °C |
| 111 | 4-Me | Me | NH | 2-Cl-Ph | 168 °C |
| 112 | - | (CH2)3 | NH | 4-Cl-Ph | 191 °C |
| 113 | - | (CH2)3 | NH | 2-Me-Ph | 174 °C |
| 114 | 4-Me | Me | NH | 3-Cl-Ph | 184 °C |
| 115 | 4-F | Me | NH | 3-Cl-Ph | 124 °C |
| 116 | 4-Me | Me | NH | 4-F-Ph | 186 °C |
| 117 | 4-Me | Me | NH | 4-Me-Ph | 157 °C |
| 118 | 4-F | Me | NH | 4-Me-Ph | 158 °C |
| 119 | 4-Me | Me | NH | 3-Me-Ph | 178 °C |
| 121 | 4-F | Me | NH | 4-Cl-Ph | 159 °C |
| 121 | 4-F | Me | NH | 4-Cl-Ph | 159 °C |
| 122 | - | Me | NH | 2.4-(Me)2-Ph | 63°C |
| 123 | - | Me | NH | 3-Cl-2-Pyr | 127°C |
| 124 | 4-Cl | Me | NH | 2-F-Ph | 120°C |
| 125 | 4-F | Me | NH | 2-F-Ph | 112°C |
| 126 | 4-Me | Me | NH | 2-F-Ph | 156°C |

On a obtenu également les composés de formule suivante :

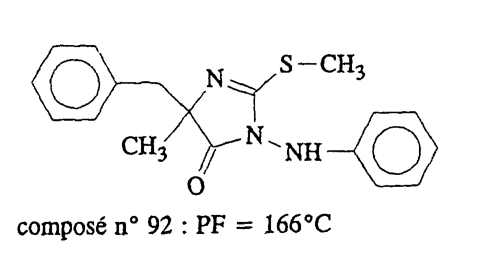

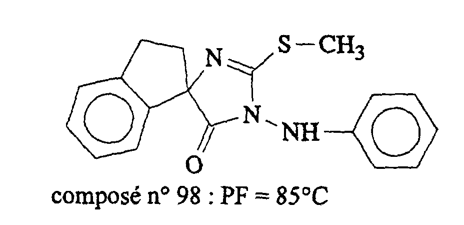

### Exemple 11: Préparation de la 4-phényl-4-méthyl-1-(phénylthio)-2-méthylthio-2-imidazoline-5-one(composé 95: PF 112°C).

Dans un tricol de 100 ml sous atmosphère inerte, on charge 0,6g (2,7 m moles) de 2-méthylthio-4-méthyl-4-phényl-2-imidazoline-5-one en solution dans 50 ml de tétrahydrofurane(THF) anhydre. On agite magnétiquement et on refroidit à 0°C( bain de glace + acétone). On ajoute 0,30 g(1 équivalent molaire) de tertiobutylate de potassium et on agite 10 min à 0°C. On coule ensuite une solution de 0,40g de chlorure de phénylsulfényle et de 10 ml de THF anhydre.
On laisse remonter le milieu à la température ambiante pendant une heure. On coule le milieu réactionnel dans 100 ml d'eau.On extrait avec 100ml d'acétate d'éthyle. On lave la phase organique 4 fois à l'eau et sèche sur sulfate de sodium.

On concentre la phase organique sous vide. On obtient un miel jaune , qui cristallise dans l'éther isopropylique après purification sur silice avec un rendement de 68%(point de fusion: 112°C).

### Exemple 12 : Test in vitro :

On étudie l'action des composés selon l'invention sur les champignons suivants responsables des maladies des céréales et autres végétaux :
Fusarium oxysporum f.sp.melonis
Rhizoctonia solani AG4
Helminthosporium gramineum
Pseudocercosporella herpotrichoïdes
Alternaria alternata
Septoria nodorum
Fusarium roseum
Pythium rostratum
Pythium vexans

Pour chaque essai, on opère de la façon suivante : un milieu nutritif constitué de pomme de terre, de glucose et de gélose (milieu PDA) est introduit en surfusion dans une série de boîte de Pétri (100 ml par boîte) après stérilisation à l'autoclave à 120°C.

Au cours du remplissage des boîtes, on injecte, dans le milieu en surfusion, une solution acétonique de la matière active, pour obtenir la concentration finale désirée.

On prend comme témoin des boîtes de Pétri analogues aux précédentes, dans lesquelles on coule des quantités similaires d'un milieu nutritif ne contenant pas de matière active.

Après 24 heures, chaque boîte est ensemencée par dépôt d'un fragment de mycelium broyé provenant d'une culture précédente du même champignon.

Les boîtes sont conservées pendant 5 jours à 20°C et on compare alors la croissance du champignon dans les boîtes contenant la matière active à tester, à celle du même champignon dans la boîte utilisée comme témoin.

On détermine ainsi pour chaque composé testé le taux d'inhibition du champignon considéré pour une dose de 20 ppm.

On obtient alors les résultats suivants :

Une bonne activité, c'est à dire un taux d'inhibition du champignon compris entre 80% et 100%, a été trouvée pour:
- les composés 9,13,16,22,26 et 34 pour Pythium rostratum et Pythium vexans.
- le composé 26 pour Fusarium oxysporum et Fusarium roseum.
- les composés 11,16,22 et 26 pour Alternaria alternata.
- les composés 11,16 et 26 pour Rhizoctonia solani.
- les composés 16 et 26 pour Pseudocercosporella herpotrichoïdes.
- les composés 11,16 et 26 pour Septoria nodorum.
- les composés 9,11,16 et 26 pour Helminthosporium gramineum.

### Exemple 13 : Test in vivo sur Plasmopara viticola (mildiou de la vigne):

On prépare, par broyage fin, une suspension aqueuse de la matière active à tester ayant la composition suivante:
- matière active: 60 mg
- agent tensioactif Tween 80, (oléate de dérivé polyoxyéthyléné du sorbitan) dilué à 10% dans l'eau : 0,3 ml
- on complète à 60 ml d'eau.

Cette suspension aqueuse est ensuite diluée par de l'eau pour obtenir la concentration désirée en matière active.

Des boutures de vigne (Vitis vinifera), variété Chardonnay, sont cultivées dans des godets. Lorsque ces plants sont âgés de 2 mois (stade 8 à 10 feuilles, hauteur de 10 à 15 cm), ils sont traités par pulvérisation au moyen de la suspension aqueuse ci-dessus.

Des plants, utilisés comme témoins sont traités par une solution aqueuse ne contenant pas la matière active.

Après séchage pendant 24 heures, on contamine chaque plant par pulvérisation d'une suspension aqueuse de spores de Plasmopara viticola obtenue à partir d' une culture de 4-5 jours, mise ensuite en suspension à raison de 100 000 unités par cm³.

Les plants contaminés sont ensuite mis en incubation pendant deux jours à 18°C environ, en atmosphère saturée d'humidité puis pendant 5 jours à 20-22°C environ sous 90-100% d'humidité relative.

La lecture se fait 7 jours après la contamination, en comparaison avec les plants témoins.

Dans ces conditions, on observe, à la dose de 1 g/l, une protection bonne (au moins 75%) ou totale avec les composés suivants : 1, 2, 9, 10, 12, 13, 15, 16, 18, 20, 22, 23, 24, 25, 30, 31, 34, 35, 37, 39 à 43, 45, 48, 55 à 58, 60, 62, 64, 68, 73, 75, 76, 83 à 85, 88 à 91, 93 à98, 101 à 108.

### Exemple 14 : Test in vivo sur Puccinia recondita (rouille du blé):

On prépare, par broyage fin, une suspension aqueuse de la matière active à tester ayant la composition suivante:
- matière active: 60 mg
- agent tensioactif Tween 80, (oléate de dérivé polyoxyéthyléné du sorbitan) dilué à 10% dans l'eau : 0,3 ml
- on complète à 60 ml d'eau.

Cette suspension aqueuse est ensuite diluée par de l'eau pour obtenir la concentration désirée en matière active.

Du blé, en godets, semé sur un substrat tourbe terre pouzzolane 50/50, est traité au stade 10 cm de hauteur par pulvérisation de la suspension aqueuse ci dessus.

Au bout de 24 heures, une suspension aqueuse de spores (100000 sp/cm³) est pulvérisée sur le blé; cette suspension a été obtenue à partir de plants contaminés. On place ensuite le blé pendant 24 heures en cellule d'incubation à environ 20°C et à 100% d'humidité relative, puis pendant 7 à 14 jours à 60% d'humidité relative.

Le contrôle de l'état des plants se fait entre le 8ème et le 15ème jour après la contamination, par comparaison avec un témoin non traité.

Dans ces conditions, on observe, à la dose de 1 g/l, une protection bonne (au moins 75%) ou totale avec les composés suivants : 2, 9, 10, 15, 18, 20 à 22 et 39, 55, 57, 64, 68, 75, 83 à 85, 88 à 90, 93, 94, 98.

### Exemple 15 : Test in vivo sur Phytophthora infestans (mildiou de la tomate) :

On prépare, par broyage fin, une suspension aqueuse de la matière active à tester ayant la composition suivante:
- matière active: 60 mg
- agent tensioactif Tween 80, (oléate de dérivé polyoxyéthyléné du sorbitan) dilué à 10% dans l'eau : 0,3 ml
- on complète à 60 ml d'eau.

Cette suspension aqueuse est ensuite diluée par de l'eau pour obtenir la concentration désirée en matière active.

Des plants de tomate (variété Marmande) sont cultivés dans des godets. Lorsque ces plants sont agés d'un mois (stade 5 à 6 feuilles, hauteur 12 à 15 cm), ils sont traités par pulvérisation de la suspension aqueuse ci dessus et à diverses concentrations du composé à tester.

Au bout de 24 heures, on contamine chaque plant par pulvérisation au moyen d'une suspension aqueuse de spores (30000 sp/cm³) de Phytophthora infestans.

Après cette contamination, les plants de tomate sont mis en incubation pendant 7 jours à 20°C environ en atmosphère saturée d'humidité.

Sept jours après la contamination, on compare les résultats obtenus dans le cas des plants traités par la matière active à tester à ceux obtenus dans le cas des plants utilisés comme témoins. Dans ces conditions, on observe, à la dose de 1 g/l, une protection bonne (au moins 75%) ou totale avec les composés suivants: 2, 9, 15, 30, 39, 45, 55, 68, 75, 84, 85, 90, 94, 98, 107, 108.

Ces résultats montrent clairement les bonnes propriétés fongicides des dérivés selon l'invention contre les maladies fongiques des plantes dues à des champignons appartenant aux familles les plus diverses telles que les Phycomycètes, les Basidiomycètes, les ascomycètes, les adelomycètes ou fungi imperfecti, en particulier le mildiou de la vigne, le mildiou de la tomate et la rouille du blé.

Pour leur emploi pratique, les composés selon l'invention sont rarement utilisés seuls. Le plus souvent ces composés font partie de compositions. Ces compositions, utilisables comme agents fongicides, contiennent comme matière active un composé selon l'invention tel que décrit précédemment en mélange avec un ou des supports solides ou liquides, acceptables en agriculture et/ou les agents tensio-actifs également acceptables en agriculture. En particulier sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... Plus généralement les composés utilisés dans l'invention peuvent être combinés à tous les additifs solides ou liquides correspondant aux techniques habituelles de la mise en formulation.

D'une façon générale, les compositions selon l'invention contiennent habituellement de 0,05 à 95 % environ (en poids) d'un composé selon l'invention (appelé par la suite matière active), un ou plusieurs supports solides ou liquides et, éventuellement, un ou plusieurs agents tensioactifs.

Par le terme "support", dans le présent exposé, on désigne une matière organique ou minérale, naturelle ou synthétique, avec laquelle le composé est combiné pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau ; alcools, notamment le butanol etc...).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque le composé et/ou le support inerte ne sont pas solubles dans l'eau et que l'agent vecteur de l'application est l'eau.

Ainsi donc, les compositions à usage agricole selon l'invention peuvent contenir les matières actives selon l'invention dans de très larges limites, allant de 0,05 % à 95 % (en poids). Leur teneur en agent tensio-actif est avantageusement comprise entre 5 % et 40 % en poids.

Ces compositions selon l'invention sont elles-mêmes sous des formes assez diverses, solides ou liquides.

Comme formes de compositions solides, on peut citer les poudres pour poudrage (à teneur en composé pouvant aller jusqu'à 100 %) et les granulés, notamment ceux obtenus par extrusion, par compactage, par imprégnation d'un support granulé, par granulation à partir d'une poudre (la teneur en composé dans ces granulés étant entre 0,5 et 80 % pour ces derniers cas), les comprimés ou tablettes effervescents.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes, les gels.

Les concentrés émulsionnables ou solubles comprennent le plus souvent 10 à 80 % de matière active, les émulsions ou solutions prêtes à l'application contenant, quant à elles, 0,001 à 20 % de matière active.

En plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités:

A partir de ces concentrés, on peut obtenir par dilution avec de l'eau des émulsions de toute concentration désirée, qui conviennent particulièrement à l'application sur les cultures.

A titre d'exemple, voici la composition de quelques concentrés émulsionnables :

### Exemple CE 1 :

- matière active 400 g/l
- dodécylbenzène sulfonate alcalin 24 g/l
- nonylphénol oxyéthylé à 10 molécules d'oxyde d'éthylène 16 g/l
- cyclohexanone 200 g/l
- solvant aromatique q.s.p.1 litre

Selon une autre formule de concentré émulsionnable, on utilise :

### Exemple CE 2

- matière active 250 g
- huile végétale époxydée 25 g
- mélange de sulfonate d'alcoylaryle et d'éther de polyglycol et d'alcools gras 100 g
- diméthylformamide 50 g
- xylène 575 g

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici une composition de suspension concentrée :

### Exemple SC 1 :

- matière active 500 g
- phosphate de tristyrylphénol polyéthoxylé 50 g
- alkylphénol polyéthoxylé 50 g
- polycarboxylate de sodium 20 g
- éthylène glycol 50 g
- huile organopolysiloxanique (antimousse) 1 g
- polysaccharide 1,5 g
- eau 316,5 g

Les poudres mouillables (ou poudre à pulvériser) sont habituellement préparées de manière qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0,1 à 10 % d'un ou plusieurs stabilisants et/ou autres additifs, comme des agents de pénétration, des adhésifs, ou des agents antimottants, colorants, etc...

Pour obtenir les poudres à pulvériser ou poudres mouillables, on mélange intimement les matières actives dans les mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables trés avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser) :

### Exemple PM 1

- matière active 50%
- alcool gras éthoxylé (agent mouillant) 2,5%
- phényléthylphénol éthoxylé (agent dispersant) 5%
- craie (support inerte) 42,5%

### Exemple PM 2 :

- matière active 10%
- alcool synthétique oxo de type ramifié, en C13 éthoxylé par 8 à 10 oxyde d'éthylène (agent mouillant) 0,75%
- lignosulfonate de calcium neutre (agent dispersant) 12%
- carbonate de calcium (charge inerte) q.s.p. 100 %

### Exemple PM 3 :

Cette poudre mouillable contient les mêmes ingrédients que dans l'exemple précédent, dans les proportions ci-après :
- matière active 75%
- agent mouillant 1,50%
- agent dispersant 8%
- carbonate de calcium (charge inerte) q.s.p. 100%

### Exemple PM 4 :

- matière active 90%
- alcool gras éthoxylé (agent mouillant) 4%
- phényléthylphénol éthoxylé (agent dispersant) 6%

### Exemple PM 5 :

- matière active 50%
- mélange de tensio-actifs anioniques et non ioniques (agent mouillant) 2,5%
- lignosulfonate de sodium (agent dispersant) 5%
- argile kaolinique (support inerte) 42,5%

Les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les composés selon l'invention peuvent être formulés sous la forme de granulés dispersibles dans l'eau également compris dans le cadre de l'invention.

Ces granulés dispersibles, de densité apparente généralement comprise entre environ 0,3 et 0,6 ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en poids du granulé) dont plus de la moitié est, par exemple, constituée par au moins un agent dispersant, essentiellement anionique, tel qu'un polynaphtalène sulfonate alcalin ou alcalino terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassage suivi d'un tamisage à la dimension de particule choisie dans les limites mentionnées ci-dessus.

De préférence, il est obtenu par extrusion, en opérant comme indiqué dans les exemples ci-après.

### Exemple GD1 : Granulés dispersibles

Dans un mélangeur, on mélange 90 % en poids de matière active et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

### Exemple GD2 : Granulés dispersibles

Dans un mélangeur, on mélange les constituants suivants :
- matière active 75%
- agent mouillant (alkylnaphtalène sulfonate de sodium) 2%
- agent dispersant (polynaphtalène sulfonate de sodium) 8%
- charge inerte insoluble dans l'eau (kaolin) 15%

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

En ce qui concerne les compositions adaptées au stockage et au transport, elles contiennent plus avantageusement de 0,5 à 95 % (en poids) de substance active.

Les compositions fongicides selon l'invention peuvent contenir des associations d'un composé de formule I avec une autre matière active, par example un fongicide. Les compositions selon l'invention sont caractérisés en ce qu'on peut les obtenir de granulés qui comprennent un composé de formule I et de poudres mouillables, de granulés ou de suspensions aqueuses qui comprennent l'autre matiére active, qui peut être un fongicide.

L'invention a également pour objets l'utilisation des composés selon l'invention pour la lutte contre les maladies fongiques des plantes par traitement préventif ou curatif de ces dernières ou de leur lieu de croissance; et

un procédé de traitement des cultures atteintes ou susceptible d'être atteintes par les maladies fongiques caractérisé en ce que l'on applique, de façon préventive ou curative, une quantité efficace d'un composé ou d'une composition selon l'invention.

Us s'appliquent avantageusement à des doses de 0,005 à 5 kg/ha, et plus spécifiquement de 0,01 à 1 kg/ha.

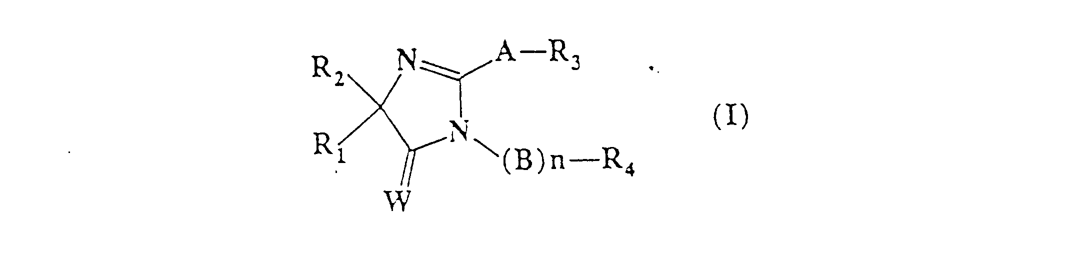

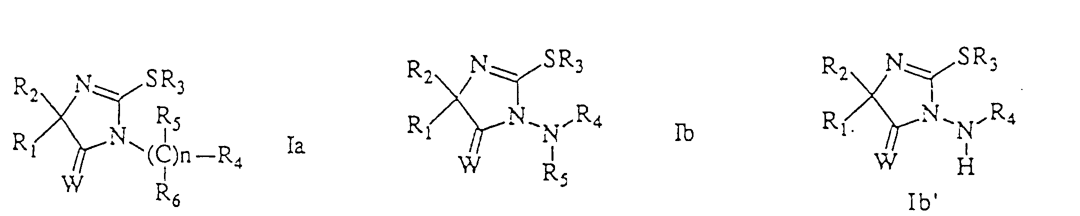

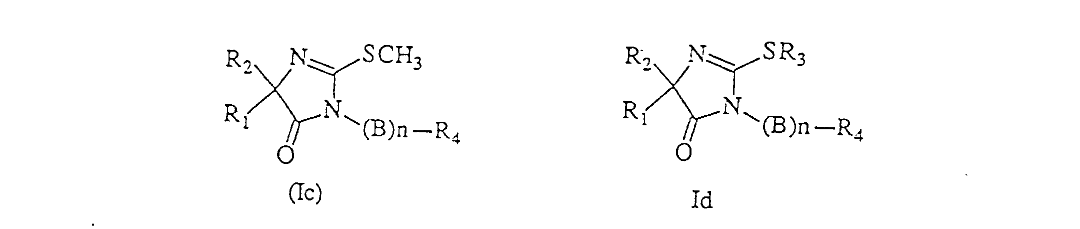

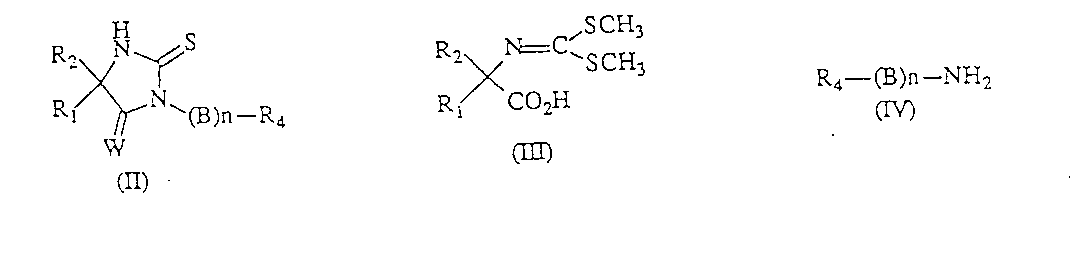

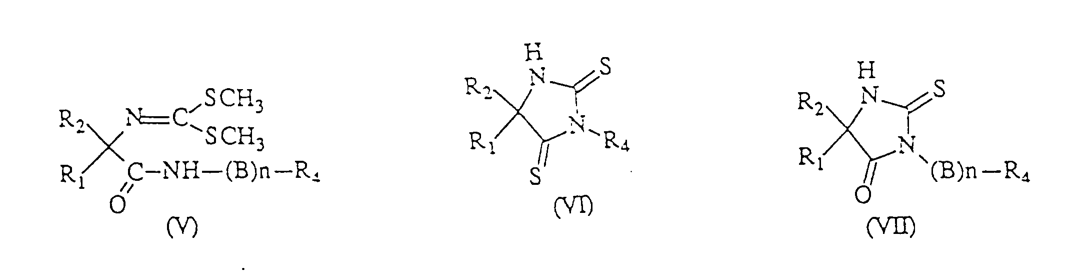

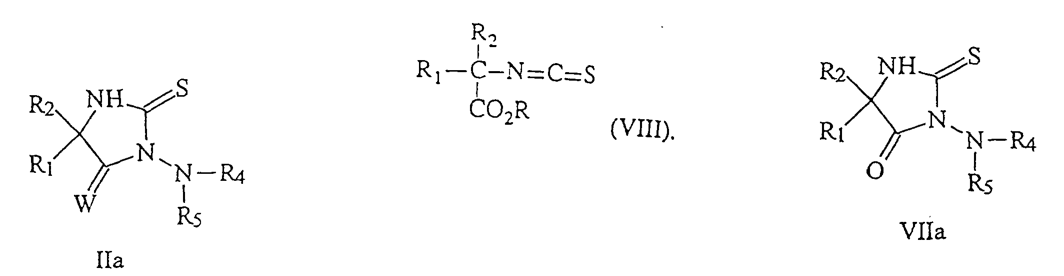

## Revendications

1. Composés à groupement imidazolinone ou imidazolinethione **caractérisés en ce qu'**ils répondent à la formule générale (I) : 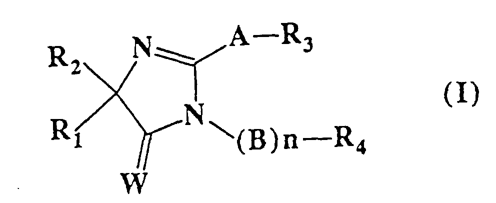 dans laquelle :
- W est l'atome de soufre ou d'oxygène ou le groupe S=O.
- A représente O ou S
- n =0 ou 1
- B représente N(R5) ou O ou S ou C(R5)(R6) ou SO2 ou C=O.
- R1 et R2, identiques ou différents représentent :
- H, à condition qu'un des 2 groupes soit différent de H, ou
- un radical alkyl ou haloalkyl de 1 à 6 atomes de carbone ou
- un radical alkoxyalkyl, alkylthioalkyl, alkylsulfonylalkyl, mono alkylaminoalkyl, alcènyl ou alcynyl de 2 à 6 atomes de carbone ou
- un radical dialkylaminoalkyl ou cycloalkyl de 3 à 7 atomes de carbone ou
- un radical aryl, comprenant phényl, naphtyl, thiényl, furyl, pyridyl, benzothiényl, benzofuryl, quinolinyl, isoquinolinyl, ou méthylène dioxyphényl, éventuellement substitué par 1 à 3 groupements choisis parmi R7 ou
- un radical arylalkyl, aryloxyalkyl, arylthioalkyl ou arylsulfonylalkyl, les termes aryl et alkyl ayant les définitions données ci-dessus ou
- R1 et R2 peuvent former, avec le carbone auquel ils sont liés sur le cycle, un carbocycle ou un hétérocycle ayant de 5 à 7 atomes, ces cycles pouvant être fusionnés avec un phényl, éventuellement substitué par 1 à 3 groupes choisis parmi R7;
- R3 représente :
- un groupe alkyl de 1 à 6 atomes de carbone ou
- un groupe alkoxyalkyl, alkylthioalkyl, alkylsulfonylalkyl, haloalkyl, cynoalkyl, thiocyanatoalkyl, oxoalkyl, alcényl ou alcynyl, de 2 à 6 atomes de carbone ou
- un groupe dialkylaminoalkyl, alkoxycarbonylalkyl, ou N-alkylcarbamoylalkyl de 3 à 6 atomes de carbone ou
- un groupe N,N-dialkylcarbamoylalkyl de 4 à 8 atomes ou
- un groupe arylalkyl, la partie alkyl étant un radical de 1 à 6 atomes de carbone et la partie aryl est phényl, naphtyl, thiényl, furyl, pyridyl, éventuellement substitué par 1 à 3 groupements choisis parmi R7;
- R4 représente :
- l'atome d'hydrogène quand n est égal à 1 ou
- un groupe alkyl de 1 à 6 atomes de carbone ou
- un groupe alkoxyalkyl, alkylthioalkyl, haloalkyl, cyanoalkyl, thiocyanatoalkyl, alcényl ou alcynyl de 2 à 6 atomes de carbone ou
- un groupe dialkylaminoalkyl, alkoxycarbonylalkyl, ou N-alkylcarbamoylalkyl de 3 à 6 atomes de carbone ou
- un groupe N,N-dialkylcarbamoylalkyl de 4 à 8 atomes de carbone ou
- un radical aryl, comprenant phényl, naphtyl, thiényl, furyl, pyridyl, pyrimidyl, pyridazinyl, pyrazinyl, benzothiényl, benzofuryl, quinolinyl, isoquinolinyl, ou méthylène dioxyphényl, éventuellement substitué par 1 à 3 groupements choisis parmi R7 ou
- un radical arylalkyl, aryloxyalkyl, arylthioalkyl ou arylsulfonylalkyl les termes aryl et alkyl ayant les définitions données ci-dessus ou
- un groupe amino disubtitué par 2 groupes identiques ou différents choisis parmi:
- un radical alkyl de 1 à 6 atomes de carbone
- un radical alkoxyalkyl, alcényl, ou alcynyl de 3 à 6 atomes de carbone
- un radical cycloalkyl de 3 à 7 atomes de carbone
- un radical arylalkyl, tel que défini ci-dessus, phényl ou naphtyl, éventuellement substitué par 1 à 3 groupements choisis parmi R7 ou
- un radical thiénylméthyl ou furfuryl
- un groupe pyrrolidino, pipéridino, morpholino ou pipérazino éventuellement substitué par alkyl de 1 à 3 atomes de carbone;
- R5 représente :
- H, sauf quand R4 est H, ou
- un radical alkyl, haloalkyl, alkylsulfonyl, haloalkylsulfonyl de 1 à 6 atomes de carbone ou
- un radical alkoxyalkyl, alkylthioakyl, acyl, alcényl, alcynyl, haloacyl, alkoxycarbonyl, haloalkoxycarbonyl, alkoxyalkylsulfonyl, cyanoalkylsulfonyl de 2 à 6 atomes de carbone ou
- un radical alkoxyalkoxycarbonyl, alkylthioalkoxycarbonyl, cyanoalkoxycarbonyl de 3 à 6 atomes de carbone ou
- le radical formyl ou
- un radical cycloalkyl, alkoxyacyl, alkylthioacyl, cyanoacyl, alcénylcarbonyl, alcynylcarbonyl de 3 à 6 atomes de carbone ou
- un radical cycloalkylcarbonyl de 4 à 8 atomes de carbone ou
- un radical phényl; arylalkylcarbonyl, notamment phénylacétyl et phényl propionyl;arylcarbonyl,notamment benzoyl, éventuellement substitué par 1 à 3 groupes parmi R7; thiénylcarbonyl; furylcarbonyl; pyridylcarbonyl; benzyloxycarbonyl; furfuryloxycarbonyl; tetrahydrofurfuryloxycarbonyl; thiénylméthoxycarbonyl; pyridylméthoxycarbonyl; phénoxycarbonyl ou phénylthiocarbonyl, le phényl étant lui-même éventuellement sustitué par 1 à 3 groupement parmi R7; alkylthiocarbonyl; haloalkylthiocarbonyl; alkoxyalkylthiocarbonyl; cyanoalkylthiocarbonyl; benzylthiocarbonyl; furfurylthiocarbonyl; tétrahydrofurfurylthiocarbonyl; thiénylméthylthiocarbonyl; pyridylméthylthiocarbonyl; ou arylsulfonyl ou
- un radical carbamoyl éventuellement mono ou disubstitué par
- un groupe alkyl ou haloalkyl de 1 à 6 atomes de carbone ou
- un groupe cycloalkyl, alcényl ou alcynyl de 3 à 6 atomes de carbone ou
- un groupe alkoxyalkyl, alkylthioalkyl ou cyanoalkyl de 2 à 6 atomes de carbone ou
- un phényl éventuellement sustitué par 1 à 3 groupement R7;
- un groupement sulfamoyl éventuellement mono ou disubstitué par
- un groupe alkyl ou haloalkyl de 1 à 6 atomes de carbone ou
- un groupe cycloalkyl, alcényl ou alcynyl de 3 à 6 atomes de carbone ou
- un groupe alkoxyalkyl, alkylthioalkyl ou cynoalkyl de 2 à 6 atomes de carbone ou
- un phényl éventuellement sustitué par 1 à 3 groupement R7;
- un groupe alkylthioalkylsulfonyl de 3 à 8 atomes de carbone ou cycloalkylsulfonyl de 3 à 7 atomes de carbone;
- R6 représente :
- l'atome d'hydrogène ou
- le groupe cyano ou
- un groupe alkyl de 1 à 6 atomes de carbone ou cycloalkyl de 3 à 7 atomes de carbone ou
- un groupe acyl ou alkoxycarbonyl de 2 à 6 atomes de carbone ou
- le groupe benzoyl éventuellement substitué par un 1 à 3 groupes R7;
- R7 représente :
- un atome d'halogène ou
- un radical alkyl, haloalkyl, alkoxy, haloalkoxy, alkylthio, haloalkylthio ou alkylsulfonyl de 1 à 6 atomes de carbone ou
- un radical cycloalkyl, halocycloalkyl, alcényloxy, alcynyloxy, alcénylthio, alcynylthio de 3 à 6 atomes de carbone ou
- le groupe nitro ou cyano ou
- un radical amino éventuellement mono ou disubstitué par un radical alkyl ou acyl de 1 à 6 atomes de carbone ou alkoxycarbonyl de 2 à 6 atomes de carbone
- un radical phényl, phénoxy ou pyridyloxy, ces radicaux étant éventuellement substitués;
et leurs formes salifiées,
à l'exclusion des composés pour lesquels lorsque R1 et R2 sont simultanément phényle ou simultanément méthyle, B est C(R5)(R6), A est le soufre, et W est O ou S;
à l'exclusion des 3 composés avec R1 = hydrogène, n = 1, B = NR5, avec R5 et R4 = méthyle, AR3 = SCH3, W = O et R2 étant soit un hydrogène, un méthyle ou un phényle;
à l'exclusion du composé dans lequel R1 est un phényl, R2, R3 et R4 sont méthyl, n est égal à zéro, et A et W sont chacun l'atome de soufre ;
à l'exclusion également des composés de formule (I) pour lesquels :
- n=0 ; et
- R1 et R2 représentent simultanément un méthyle ; et
- A est un atome de soufre ; et
- R4 est un phényle substitué par 2 groupements :
- l'un choisi parmi les groupes cyano, nitro, ou un atome d'halogène ;
- l'autre choisi parmi un groupe trifluorométhyle ou un atome d'halogène ;
et à l'exclusion des composés suivants :
- la 3-[(4-chlorophenyl)sulfonyl]-3,5-dihydro-2-methoxy-5-methyl-5(1-methylethyl)-4H-imidazol-4-one ;
- la 2-(éthoxycarbonylméthylthio)-3-acétyl-5,5-diphenyl-3,5-dihydro-4*H*-imidazol-4-one
- la 2-butoxy-5-ethyl-3,5-dihydro-3-methyl-5-phenyl-4H-imidazol-4-one;
- la 2-methoxy-5-methyl-3,5-dihydro-3-(1-phenylethyl)-4H-imidazol-4-one;
- la 2-methylthio-5,5-dimethyl-3,5-dihydro-3-phenyl-4H-imidazol-4-one;
- la 2-(methylthio)-3,5,5-triphenyl-3,5-dihydro-4*H*-imidazol-4-one;
- la 3-methyl-2-(methylthio)-1,3-diazaspiro[4,5]dec-1-ene-4-one ;
- la 3-methyl-2-(methylthio)-1,3-diazaspiro[4,5]dec-1-ene-4-thione.

2. Composés selon la revendication 1 caractérisés en ce A est l'atome de soufre.

3. Composés selon la revendication 1 caractérisés en ce W est l'atome d'oxygène.

4. Composés selon la revendication 2 **caractérisés en ce qu'**ils répondent à la formule générale : 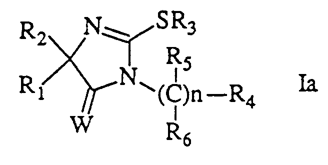 dans laquelle W, n, R1 à R6 ont la même signification que dans la revendication 1.

5. Composés selon la revendication 2 **caractérisés en ce qu'**ils répondent à la formule générale : 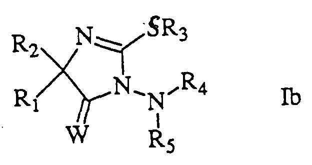 dans laquelle W, R1 à R5 ont la même signification que dans la revendication 1.

6. Composés selon la revendication 2 **caractérisés en ce qu'**ils répondent à la formule générale : 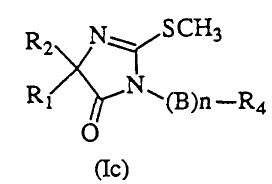 dans laquelle B, n, R1 à R4 ont la même signification que dans la revendication 1.

7. Composés selon la revendication 2 **caractérisés en ce qu'**ils répondent à la formule générale : 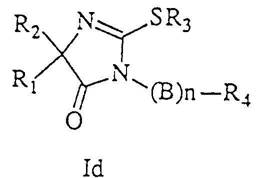 dans laquelle B, n, R1 à R4 ont la même signification que dans la revendication 1.

8. Composés selon l'une des revendications 1 à 7 **caractérisés en ce que** R5 est l'atome d'hydrogène.

9. Composés selon la revendication 5 **caractérisés en ce que** R5 est l'atome d'hydrogène (composé Ib').

10. Composés selon l'une des revendications 1 à 9 **caractérisés en ce que** R1 et R2 sont différents de l'atome d'hydrogène.

11. Composés selon l'une des revendications 1 à 10 **caractérisés en ce que** R2 est un groupe alkyl de 1 à 3 atomes de carbone.

12. Composés selon l'une des revendications 1 à 11 **caractérisés en ce que** R1 est un phényl, éventuellement substitué par un groupe R7.

13. Composés selon l'une des revendications 1 à 12 **caractérisés en ce que** R3 est un groupe alkyl de 1 à 3 atomes de carbone.

14. Composés selon l'une des revendications 1 à 13 **caractérisés en ce que** R4 est un phényl, eventuellement substitué par un groupe R7.

15. Composés selon l'une des revendications 1 à 14 **caractérisés en ce que** R3 est un radical méthyl.

16. Procédé de préparation (procédé A) des composés de formule (I) selon la revendication 2, **caractérisé en ce que** l'on effectue une S-alkylation des 2-thiohydantoïnes de formule (II) avec un composé de formule R3X, en présence de base et de solvant, selon le schéma : 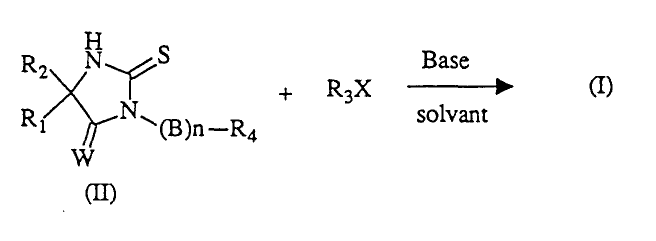 dans lequel R1,R2,R3,R4,B et W ont la signification de la revendication 1 et X est l'atome de chlore, de brome, d'iode, ou le groupe sulfate, ou un groupe alkylsulfonyloxy ou arylsulfonyloxy.

17. Procédé de préparation des composés 2-méthylthio-2-imidazoline-5-ones de formule (Ic)(procédé B) selon la revendication 6, **caractérisé en ce que** l'on cyclise, par simple chauffage dans un solvant aromatique à reflux, un composé de formule (V) selon le schéma : 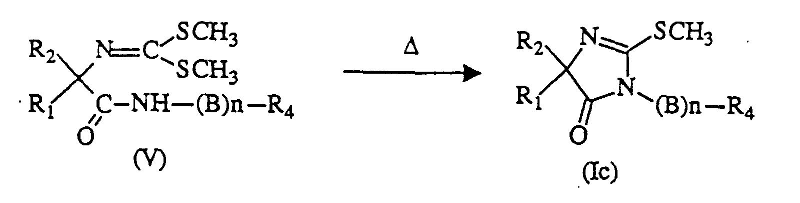 dans lequel, R1, R2, R4, B et n ont la même signification que dans la revendication 1.

18. Procédé de préparation des composés de formule (V)(procédé B) selon la revendication 17, **caractérisé en ce que** l'on effectue une condensation d'un iminodithiocarbonate de formule (III) avec une hydrazine de formule (IV) selon le schéma : 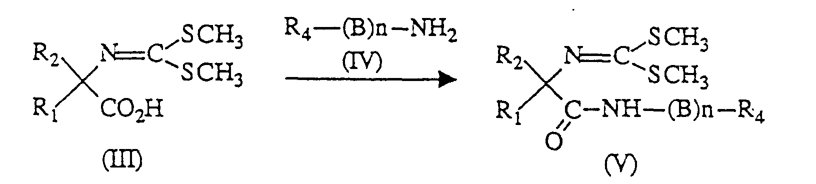 dans lequel :
- R1, R2, R4 ont la même signification que dans la revendication 1
- n égale 1 et B représente NR5
- R5 a la même signification que dans la revendication 1 ;
l'acide (III) devant être activé pour cette condensation sous forme de chlorure d'acide, de dicyclohexylisourée à l'aide de dicyclohexylcarbodiimide ou d'imidazolide à l'aide de carbonyldiimidazole.

19. Procédé de préparation des composés de formule (Ib)(procédé C1), selon le schéma : 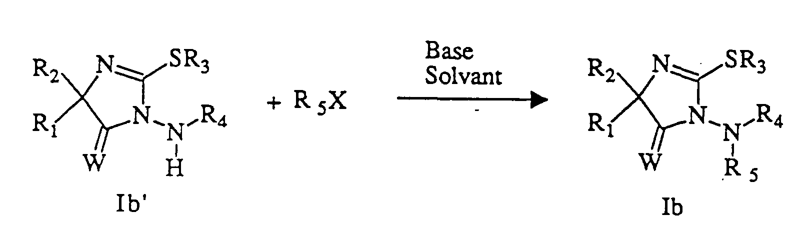 **caractérisé en ce que** l'on fait réagir, dans un solvant en présence de base, un composé de formule R5X sur un composé de formule (Ib) selon la revendication 5, dans lequel le groupe R5 est l'atome d'hydrogène selon la revendication 9 (composé (Ib')), les groupements R1 à R4 ont la signification donnée à la revendication 1, le groupe R5 est un groupe alkyl, alcoxycarbonyl, acyl, aroyl, alkylsulfonyl, arylsulfonyl, carbamoyl ou sulfamoyl et X représente un groupe partant tel que un halogène, le groupe sulfate, un phénoxy éventuellement substitué, R5O quand R5 est acyl, ou un groupe alkylsulfonyloxy ou arylsulfonyloxy ou un groupe non partant tel que isocyanate ou isothiocyanate.

20. Procédé de préparation des composés de formule (Id)(procédé C2), selon le schéma : 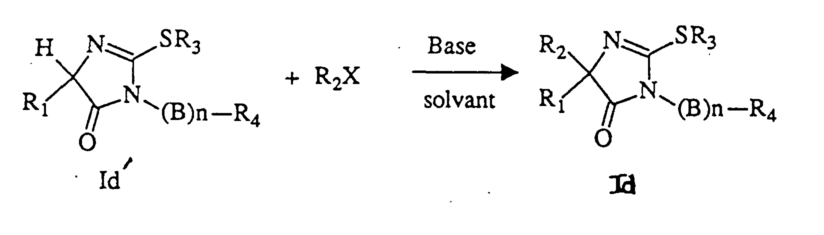 **caractérisé en ce que** l'on fait réagir un composé de formule (Id), selon la revendication 7, dans laquelle R2 est l'atome d'hydrogène (composé (Id')) avec un composé de formule R2X dans un solvant et en présence de base, à une température comprise entre -30°C et +80°C, X étant un atome de chlore, de brome, ou d'iode et R1 à R4, B, et n ayant la même signification que dans la revendication 1.

21. Procédé selon la revendication 20, **caractérisé en ce que** la base est choisie parmi le groupe comprenant un alcoolate, un hydrure métallique ou un amidure et le solvant est choisi parmi le groupe comprenant les éthers, les éthers cycliques, le diméthylformamide, le diméthylsulfoxyde, les solvants aromatiques.

22. Procédé de préparation des composés de formule (I)(procédé D) selon la revendication 1 dans lesquels W représente le groupe S=O, **caractérisé en ce que** l'on effectue une S-oxydation d'un composé de formule (I) dans lequel W est l'atome de soufre, cette oxydation de 2-imidazoline-5-thiones étant réalisée avec une quantité stoechiométrique de peroxydes dans un solvant à une température comprise entre - 20°C et + 20°C.

23. Procédé selon la revendication 22 **caractérisé en ce que** le peroxyde est un peracide et le solvant est choisi dans le groupe comprenant le chloroforme et le chlorure de méthylène.

24. Composés utiles notamment comme intermédiaires dans la préparation des composés selon l'une des revendications 1 à 15 **caractérisés en ce qu'**ils répondent aux formules IIa, III, V, et VIIa: 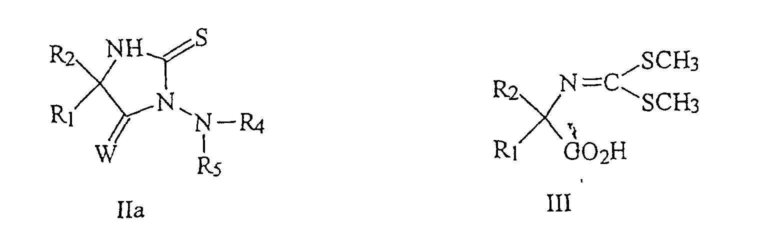 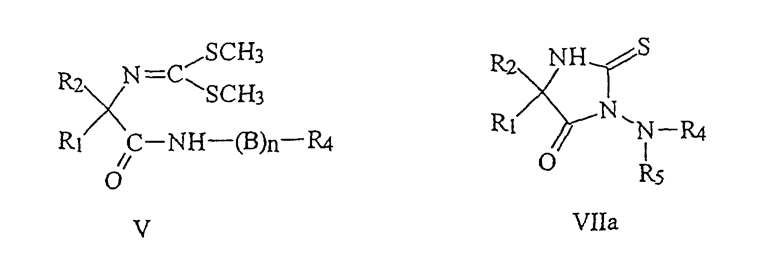 dans lesquelles W, R1, R2, R4 et R5, n et B sont tels que définis dans la revendication 1, R4 étant autre que l'atome d'hydrogène, à l'exclusion des composés selon formule IIa avec:
W=S
R¹ = methyl, R² = phenyl, R⁵ = hydrogène et R⁴ = methyl ou phenyl;
W = S,
R¹ = ethyl, R² = phenyl, R⁴ = methyl et R⁵ hydrogène;
W = S,
R¹ = methyl, R² = p-tolyl, et
R⁴ = methyl, R⁵ = hydrogène ou methyl ou
R⁴ = phenyl, R⁵ = hydrogène;
W = S,
R¹ et R² = phenyl, R⁴ = methyl et R⁵ = H,
et à l'exclusion des composés selon la formule VIIa avec:
R₄ = methyl, R₅ = methyl, R₁ = hydrogen
et R₂ = methyl ou phenyl.

25. Composés selon la revendication 24 **caractérisés en ce que**
R⁵ est l'atome d'hydrogène,
R² représente un groupe alkyl de 1 à 3 atomes de carbone,
R¹ représente le noyau phényl, éventuellement substitué par R⁷,
R⁴ représente le noyau phényl, éventuellement substitué par R⁷, et
R⁷ est tel que défini dans la revendication 1.

26. Composés selon la revendication 24 ou 25, **caractérisés en ce qu'**ils répondent à la formule VIIa.

27. Composés selon la revendication 25 ou 26 **caractérisés en ce que** R² représente le groupe méthyl.

28. Composés selon la revendication 25, 26 ou 27 **caractérisés en ce que**
R¹ représente le noyau phényl.

29. Procédé de préparation des 2-thiohydantoïnes de formule (VII) (procédé F): 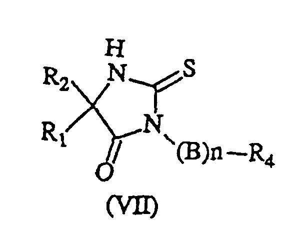 **caractérisé en ce que** l'on fait réagir, avant cyclisation, un composé de formule (IV) R4(B)nNH2 sur des isothiocyanates dérivés des aminoacides de formule (VIII): 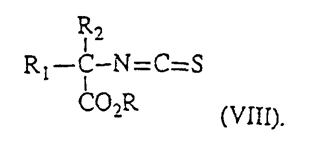 dans lesquelles,R1,R2,R4 ont la même signification que dans la revendication 1, n = 1 et B représente N(R5).

30. Procédé selon la revendication 29 **caractérisé en ce que** la cyclisation est réalisée thermiquement par chauffage du mélange des réactifs à une température comprise entre 110 et 180 °C dans un solvant aromatique tel que le toluène, le xylène, les chlorobenzènes.

31. Procédé selon la revendication 29 **caractérisé en ce que** la cyclisation est réalisée avec un équivalent de base, dans un solvant et à température comprise entre -10 et +80°C, le milieu étant par la suite neutralisé à température ambiante.

32. Procédé selon la revendication 31 **caractérisé en ce que** la base est choisie parmi le groupe comprenant un alcoolate alcalin, un hydroxyde alcalin ou une amine tertiaire; le solvant est choisi parmi le groupe comprenant les éthers, les éthers cycliques, les alcools, les esters, le DMF, le DMSO.

33. Procédé (procédé G) selon les revendications 29 et 31 **caractérisé en ce que**, avant d'aboutir aux dérivés 2-thiohydantoïnes par neutralisation en milieu acide, on engage un halogénure d'alkyl R3X pour former le composé de formule Id, X étant un atome de chlore, brome ou iode ou un groupe alkylsulfonyloxy ou arylsulfonyloxy et R3 ayant la même signification que dans la revendication 1.

34. Compositions fongicides comprenant, en association avec un ou des supports solides ou liquides acceptable en agriculture et/ou des agents tensio-actifs également acceptables en agriculture, une (ou plusieurs) matiére active qui est un composé de formule I tel que défini dans l'une des revendications 1 à15.

35. Compositions fongicides selon la revendication 34, **caractérisées en ce qu'**elles contiennent de 0,5% à 95% en poids de composés selon l'une des revendications 1 à 15.

36. Compositions fongicides selon la revendication 34 ou 35 **caractérisés en ce qu'**elles contiennent des associations d'un composé de formule I avec une autre matière active.

37. Compositions selon la revendication 36 **caractérisés en ce que** l'autre matière active est un fongicide.

38. Compositions selon la revendication 36 ou 37 **caractérisés en ce qu'**on peut les obtenir de granulés qui comprennent un composé de formule I et de poudres mouillables, de granulés ou de suspensions aqueuses qui comprennent l'autre matiére active.

39. Compositions selon la revendication 38 **caractérisés en ce que** l'autre matière active est un fongicide.

40. Procédé de traitement des cultures atteintes ou susceptibles d'être atteintes par les maladies fongiques **caractérisé en ce que** l'on applique, de façon préventive ou curative, une quantité efficace d'un composé selon l'une des revendications 1 à 15 ou d'une composition selon les revendications 34 à 39.

41. Procédé de traitement selon la revendication 40, **caractérisé en ce que** la dose efficace est comprise entre 0,005 et 5 kg/ha.

42. Procédé de traitement selon la revendication 41, **caractérisé en ce que** la dose efficace est comprise entre 0,01 et 1 kg/ha.

## Patentansprüche

1. Imidazolinon- oder Imidazolinthionverbindungen, **dadurch gekennzeichnet, dass** diese der allgemeinen Formel (I) : 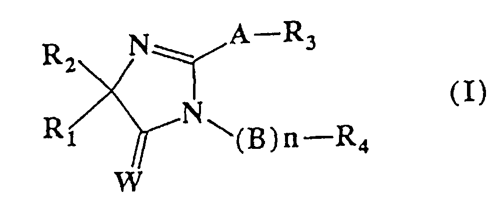 entsprechen, in welcher:
- W ein Schwefel- oder Sauerstoffatom oder die Gruppe S=O ist,
- A für O oder S steht,
- n = 0 oder 1 ist,
- B für N(R5) oder O oder S oder C(R5)(R6) oder SO2 oder C=O steht,
- R1 und R2, welche identisch oder verschieden sein können, stehen für:
- H, unter der Bedingung, dass eine der 2 Gruppen von H verschieden ist, oder
- eine Alkyl- oder Halogenalkylgruppe mit 1 bis 6 Kohlenstoffatomen oder
- eine Alkoxyalkyl-, Alkylthioalkyl-, Alkylsulfonylalkyl-, Monoalkylaminoalkyl-, Alkenyl- oder Alkinylgruppe mit 2 bis 6 Kohlenstoffatomen oder
- eine Dialkylaminoalkyl- oder Cycloalkylgruppe mit 3 bis 7 Kohlenstoffatomen oder
- eine Arylgruppe, umfassend Phenyl, Naphtyl, Thienyl, Furyl, Pyridyl, Benzothienyl, Benzofuryl, Chinolinyl, Isochinolinyl oder Methylendioxyphenyl, gegebenenfalls substituiert mit 1 bis 3 Gruppen, die ausgewählt sind aus R7, oder
- eine Arylalkyl-, Aryloxyalkyl-, Arylthioalkyl- oder Arylsulfonylalkylgruppe, wobei die Begriffe Aryl und Alkyl die nachstehend angegebenen Definitionen aufweisen,
- R1 und R2 zusammen mit dem Kohlenstoff, an welchem sie an den Ring gebunden sind, einen Kohlenstoffring oder einen Heterocyclus mit 5 bis 7 Atomen bilden können, wobei diese Ringe mit einem Phenyl verbunden sein können, das gegebenenfalls mit 1 bis 3 Gruppen, die aus R7 ausgewählt sind, substituiert sein kann;
- R3 steht für:
- eine Alkylgruppe mit 1 bis 6 Kohlenstoffatomen oder
- eine Alkoxyalkyl-, Alkylthioakyl-, Alkylsulfonylalkyl-, Halogenalkyl-, Cynoalkyl-, Thiocyanatoalkyl-, Oxoalkyl-, Alkenyl- oder Alkinylgruppe mit 2 bis 6 Kohlenstoffatomen oder
- eine Dialkylaminoalkyl-, Alkoxycarbonylalkyl- oder N-Alkylcarbamoylalkylgruppe mit 3 bis 6 Kohlenstoffatomen oder
- eine N,N-Dialkylcarbamoylalkylgruppe mit 4 bis 8 Atomen oder
- eine Arylalkylgruppe, wobei der Alkylanteil eine Gruppe mit 1 bis 6 Kohlenstoffatomen ist und der Arlyanteil Phenyl, Naphtyl, Thienyl, Furyl, Pyridyl, gegebenenfalls substituiert mit 1 bis 3 Gruppen, die ausgewählt sind aus R7, ist;
- R4 steht für:
- das Wasserstoffatom, wenn n gleich 1 ist, oder
- eine Alkylgruppe mit 1 bis 6 Kohlenstoffatomen oder
- eine Alkoxyalkyl-, Alkylthioalkyl-, Halogenalkyl-, Cyanoalkyl-, Thiocyanatoalkyl-, Alkenyl- oder Alkinylgruppe mit 2 bis 6 Kohlenstoffatomen oder
- eine Dialkylaminoalkyl-, Alkoxycarbonylalkyl- oder N-Alkylcarbamoylalkylgruppe mit 3 bis 6 Kohlenstoffatomen oder
- eine N,N-Dialkylcarbamoylalkylgruppe mit 4 bis 8 Kohlenstoffatomen oder
- eine Arylgruppe, umfassend Phenyl, Naphtyl, Thienyl, Furyl, Pyridyl, Pyrimidyl, Pyridazinyl, Pyrazinyl, Benzothienyl, Benzofuryl, Chinolinyl, Isochinolinyl oder Methylendioxyphenyl, gegebenenfalls substituiert mit 1 bis 3 Gruppen, die ausgewählt sind aus R7, oder
- eine Arylalkyl-, Aryloxyalkyl-, Arylthioalkyl- oder Arylsulfonylalkylgruppe, wobei die Begriffe Aryl und Alkyl die nachstehend angegebenen Definitionen aufweisen, oder
- eine Aminogruppe, zweifach substituiert mit 2 identischen oder unterschiedlichen Gruppen, die ausgewählt sind aus:
- einer Alkylgruppe mit 1 bis 6 Kohlenstoffatomen
- einer Alkoxyalkyl-, Alkenyl- oder Alkinylgruppe mit 3 bis 6 Kohlenstoffatomen
- einer Cycloalkylgruppe mit 3 bis 7 Kohlenstoffatomen
- einer Arylalkylgruppe, wie sie nachstehend definiert ist, Phenyl oder Naphtyl, gegebenenfalls substituiert mit 1 bis 3 Gruppen, die ausgewählt sind aus R7, oder
- einer Thienylmethyl- oder Furfurylgruppe
- eine Pyrrolidino-, Piperidino-, Morpholino- oder Piperazinogruppe, gegebenenfalls substituiert mit einem Alkyl mit 1 bis 3 Kohlenstoffatomen;
- R5 steht für:
- H, außer wenn R4 H ist, oder
- eine Alkyl-, Halogenakyl-, Alkylsulfonyl-, Halogenalkylsulfonylgruppe mit 1 bis 6 Kohlenstoffatomen oder
- eine Alkoxyalkyl-, Alkylthioalkyl-, Acyl-, Alkenyl-, Alkinyl-, Halogenacyl-, Alkoxycarbonyl-, Halogenalkoxycarbonyl-, Alkoxyalkylsulfonyl-, Cyanoalkylsulfonylgruppe mit 2 bis 6 Kohlenstoffatomen oder
- eine Alkoxyalkoxycarbonyl-, Alkylthioalkoxycarbonyl-, Cyanoalkoxycarbonylgruppe mit 3 bis 6 Kohlenstoffatomen oder
- die Formylgruppe oder
- eine Cycloalkyl-, Alkoxyacyl-, Alkylthioacyl-, Cyanoacyl-, Alkenylcarbonyl-, Alkinylcarbonylgruppe mit 3 bis 6 Kohlenstoffatomen oder
- eine Cycloalkylcarbonylgruppe mit 4 bis 8 Kohlenstoffatomen oder
- eine Phenylgruppe; Arylalkylcarbonyl, insbesondere Phenylacetyl und Phenylpropionyl; Arylcarbonyl, insbesondere Benzoyl, gegebenenfalls substituiert mit 1 bis 3 Gruppen aus R7; Thienylcarbonyl; Furylcarbonyl; Pyridylcarbonyl; Benzyloxycarbonyl; Furfuryloxycarbonyl; Tetrahydrofurfuryloxycarbonyl; Thienylmethoxycarbonyl; Pyridylmethoxycarbonyl; Phenoxycarbonyl oder Phenylthiolcarbonyl, wobei das Phenyl selbst gegebenenfalls mit 1 bis 3 Gruppen aus R7 substituiert ist; Alkylthiolcarbonyl; Halogenalkylthiolcarbonyl; Alkoxyalkylthiolcarbonyl; Cyanoalkylthiolcarbonyl; Benzylthiolcarbonyl; Furfurylthiolcarbonyl; Tetrahydrofurfurylthiolcarbonyl; Thienylmethylthiolcarbonyl; Pyridylmethylthiolcarbonyl; oder Arylsulfonyl oder
- eine Carbamoylgruppe, gegebenenfalls mono- oder disubstituiert mit:
- einer Alkyl- oder Halogenalkylgruppe mit 1 bis 6 Kohlenstoffatomen oder
- einer Cycloalkyl-, Alkenyl- oder Alkinylgruppe mit 3 bis 6 Kohlenstoffatomen oder
- einer Alkoxyalkyl-, Alkylthioalkyl- oder Cyanoalkylgruppe mit 2 bis 6 Kohlenstoffatomen oder
- einem Phenyl, gegebenenfalls substituiert mit 1 bis 3 Gruppen R7;
- eine Sulfamoylgruppe, gegebenenfalls mono- oder disubstituiert mit:
- einer Alkyl- oder Halogenalkylgruppe mit 1 bis 6 Kohlenstoffatomen oder
- einer Cycloalkyl-, Alkenyl- oder Alkinylgruppe mit 3 bis 6 Kohlenstoffatomen oder
- einer Alkoxyalkyl-, Alkylthioalkyl- oder Cynoalkylgruppe mit 2 bis 6 Kohlenstoffatomen oder
- einem Phenyl, gegebenenfalls substituiert mit 1 bis 3 Gruppen R7;
- eine Alkylthioalkylsulfonylgruppe mit 3 bis 8 Kohlenstoffatomen oder Cycloalkylsulfonyl mit 3 bis 7 Kohlenstoffatomen;
- R6 steht für:
- das Wasserstoffatom oder
- die Cyanogruppe oder
- eine Alkylgruppe mit 1 bis 6 Kohlenstoffatomen oder Cycloalkyl mit 3 bis 7 Kohlenstoffatomen oder
- eine Acyl- oder Alkoxycarbonylgruppe mit 2 bis 6 Kohlenstoffatomen oder
- die Benzoylgruppe, gegebenenfalls substituiert mit 1 bis 3 Gruppen R7;
- R7 steht für:
- ein Halogenatom oder
- eine Alkyl-, Halogenakyl-, Alkoxy-, Halogenalkoxy-, Alkylthio-, Halogenalkylthio- oder Alkylsulfonylgruppe mit 1 bis 6 Kohlenstoffatomen oder
- eine Cycloalkyl-, Halogencycloalkyl-, Alkenyloxy-, Alkinyloxy-, Alkenylthio-, Alkinylthiogruppe mit 3 bis 6 Kohlenstoffatomen oder
- die Nitro- oder Cyanogruppe oder
- eine Aminogruppe, gegebenenfalls mono- oder disubstituiert mit einer Alkyl- oder Acylgruppe mit 1 bis 6 Kohlenstoffatomen oder Alkoxycarbonyl mit 2 bis 6 Kohlenstoffatomen
- eine Phenyl-, Phenoxy- oder Pyridyloxygruppe, wobei die Gruppen gegebenenfalls substituiert sind;
und deren Salzformen,
ausschließlich der Verbindungen, für welche, wenn R1 und R2 gleichzeitig Phenyl oder gleichzeitig Methyl sind, B C(R5)(R6) ist, A Schwefel ist und W O oder S ist;
ausschließlich der 3 Verbindungen mit R1 = Wasserstoff, n = 1, B = NR5 mit R5 = R4 = Methyl, AR3 = SCH3, W = O und wobei R2 ein Wasserstoff, ein Methyl oder ein Phenyl ist;
ausschließlich der Verbindungen, in welcher R1 ein Phenyl ist, R2, R3 und R4 Methyl sind, n gleich Null ist und A und W jeweils ein Schwefelatom sind;
ausschließlich ebenfalls der Verbindungen der Formel (I), für welche gilt:
- n = 0; und
- R1 und R2 stehen gleichzeitig für ein Methyl; und
- A ist ein Schwefelatom; und
- R4 ist ein Phenyl, das mit 2 Gruppen substituiert ist:
- der einen, ausgewählt aus der Cyanogruppe, der Nitrogruppe oder einem Halogenatom;
- der anderen, ausgewählt aus einer Trifluormethylgruppe oder einem Halogenatom;
und ausschließlich der folgenden Verbindungen:
- 3-[(4-Chlorphenyl)sulfonyl]-3,5-dihydro-2-methoxy-5-methyl-5(1-methylethyl)-4H-imidazol-4-on;
- 2-(Ethoxycarbonylmethylthio)-3-acetyl-5,5-diphenyl-3,5-dihydro-4*H*-imidazol-4-on;
- 2-Butoxy-5-ethyl-3,5-dihydro-3-methyl-5-phenyl-4H-imidazol-4-on;
- 2-Methoxy-5-methyl-3,5-dihydro-3-(1-phenylethyl)-4H-imidazol-4-on;
- 2-Methylthio-5,5-dimethyl-3,5-dihydro-3-phenyl-4H-imidazol-4-on;
- 2-(Methylthio)-3,5,5-triphenyl-3,5-dihydro-4H-imidazol-4-on;
- 3-Methyl-2-(methylthio)-1,3-diazaspiro[4,5]dec-1-en-4-on;
- 3-Methyl-2-(methylthio)-1,3-diazaspiro[4,5]dec-1-en-4-thion.

2. Verbindungen nach Anspruch 1, **dadurch gekennzeichnet, dass** A ein Schwefelatom ist.

3. Verbindungen nach Anspruch 1, **dadurch gekennzeichnet, dass** W ein Sauerstoffatom ist.

4. Verbindungen nach Anspruch 2, **dadurch gekennzeichnet, dass** diese der folgenden allgemeinen Formel: 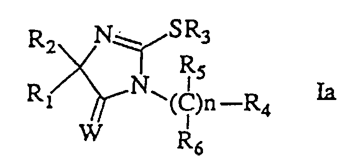 entsprechen, in welcher W, n, R1 bis R6 dieselbe Bedeutung haben wie in Anspruch 1.

5. Verbindungen nach Anspruch 2, **dadurch gekennzeichnet, dass** diese der folgenden allgemeinen Formel: 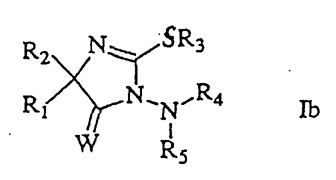 entsprechen, in welcher W, R1 bis R5 dieselbe Bedeutung haben wie in Anspruch 1.

6. Verbindungen nach Anspruch 2, **dadurch gekennzeichnet, dass** diese der folgenden allgemeinen Formel: 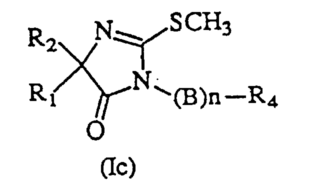 entsprechen, in welcher B, n, R1 bis R4 dieselbe Bedeutung haben wie in Anspruch 1.

7. Verbindungen nach Anspruch 2, **dadurch gekennzeichnet, dass** diese der folgenden allgemeinen Formel: 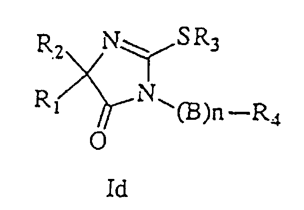 entsprechen, in welcher B, n, R1 bis R4 dieselbe Bedeutung haben wie in Anspruch 1.

8. Verbindungen nach einem der Ansprüche 1 bis 7, **dadurch gekennzeichnet, dass** R5 ein Wasserstoffatom ist.

9. Verbindungen nach Anspruch 5, **dadurch gekennzeichnet, dass** R5 ein Wasserstoffatom ist (Verbindung Ib').

10. Verbindungen nach einem der Ansprüche 1 bis 9, **dadurch gekennzeichnet, dass** R1 und R2 von einem Wasserstoffatom verschieden sind.

11. Verbindungen nach einem der Ansprüche 1 bis 10, **dadurch gekennzeichnet, dass** R2 eine Alkylgruppe mit 1 bis 3 Kohlenstoffatomen ist.

12. Verbindungen nach einem der Ansprüche 1 bis 11, **dadurch gekennzeichnet, dass** R1 ein Phenyl ist, welches gegebenenfalls mit einer Gruppe R7 substituiert ist.

13. Verbindungen nach einem der Ansprüche 1 bis 12, **dadurch gekennzeichnet, dass** R3 eine Alkylgruppe mit 1 bis 3 Kohlenstoffatomen ist.

14. Verbindungen nach einem der Ansprüche 1 bis 13, **dadurch gekennzeichnet, dass** R4 ein Phenyl ist, das gegebenenfalls mit einer Gruppe R7 substituiert ist.

15. Verbindungen nach einem der Ansprüche 1 bis 14, **dadurch gekennzeichnet, dass** R3 eine Methylgruppe ist.

16. Verfahren zur Herstellung (Verfahren A) von Verbindungen der Formel (I) nach Anspruch 2, **dadurch gekennzeichnet, dass** man eine S-Alkylierung von 2-Thiohydantoinen der Formel (II) mit einer Verbindung der Formel R3X in Gegenwart einer Base und eines Lösungsmittels nach dem folgenden Reaktionsschema: 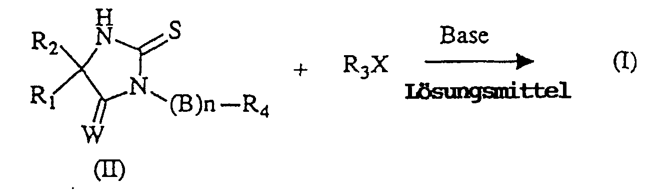 ausführt, in welchem R1, R2, R3, R4, B und W die Bedeutung aus Anspruch 1 haben und X das Chlor-, Brom- oder Iodatom oder die Sulfatgruppe oder eine Alkylsulfonyloxy- oder Arylsulfonyloxygruppe ist.

17. Verfahren zur Herstellung von 2-Methylthio-2-imidazolin-5-on-Verbindungen der Formel (Ic) (Verfahren B) nach Anspruch 6, **dadurch gekennzeichnet, dass** man durch einfaches Erwärmen in einem aromatischen Lösungsmittel unter Rückfluss eine Verbindung der Formel (V) nach dem folgenden Reaktionsschema: 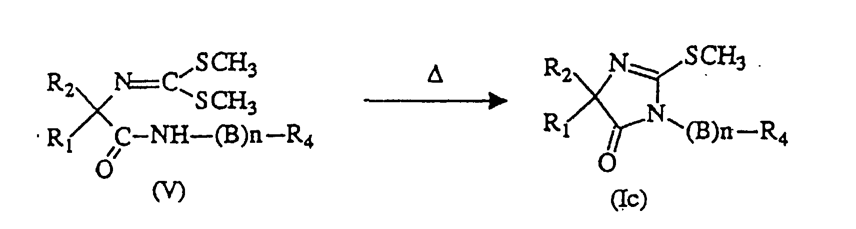 cyclisiert, in welchem R1, R2, R4, B und n dieselbe Bedeutung wie in Anspruch 1 haben.

18. Verfahren zur Herstellung von Verbindungen der Formel (V) (Verfahren B) nach Anspruch 17, **dadurch gekennzeichnet, dass** man eine Kondensation eines Iminodithiocarbonats der Formel (III) mit einem Hydrazin der Formel (IV) nach dem folgenden Reaktionsschema: 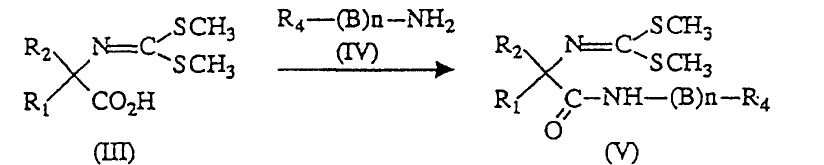 bewirkt, in welchem:
- R1, R2, R4 dieselbe Bedeutung wie in Anspruch 1 haben
- n gleich 1 ist und B für NR5 steht
- R5 dieselbe Bedeutung wie in Anspruch 1 hat;
wobei die Säure (III) für diese Kondensation in Form eines Säurechlorids, eines Dicyclohexylisoharnstoffs mit Hilfe von Dicyclohexylcarbodiimid oder eines Imidazolids mit Hilfe von Carbonyldiimidazol aktiviert werden musste.

19. Verfahren zur Herstellung von Verbindungen der Formel (Ib) (Verfahren C1) nach dem folgenden Reaktionsschema: 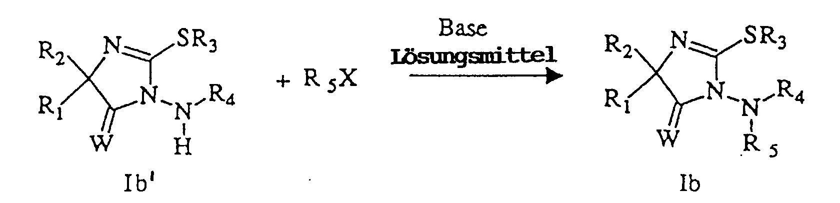 **dadurch gekennzeichnet, dass** man in einem Lösungsmittel in Gegenwart einer Base eine Verbindung der Formel R5X mit einer Verbindung der Formel (Ib) nach Anspruch 5, in welcher die Gruppe R5 nach Anspruch 9 ein Wasserstoffatom ist (Verbindung (Ib')), die Gruppen R1 bis R4 die in Anspruch 1 angegebene Bedeutung aufweisen, reagieren lässt, wobei die Gruppe R5 eine Alkyl-, Alkoxycarbonyl-, Acyl-, Aroyl-, Alkylsulfonyl-, Arylsulfonyl-, Carbamoyl- oder Sulfamoylgruppe ist und X für eine Abgangsgruppe wie z.B. ein Halogen, die Sulfatgruppe, ein Phenoxy, das gegebenenfalls substituiert ist, R50, wenn R5 Acyl ist, oder eine Alkylsulfonyloxy- oder Arylsulfonyloxygruppe oder eine Nichtabgangsgruppe wie z.B. Isocyanat oder Isothiocyanat steht.

20. Verfahren zur Herstellung von Verbindungen der Formel (Id) (Verfahren C2) nach dem folgenden Reaktionsschema: 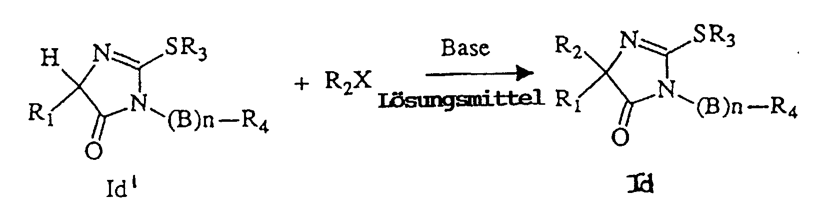 **dadurch gekennzeichnet, dass** man eine Verbindung der Formel (Id) nach Anspruch 7, in welcher R2 ein Wasserstoffatom ist (Verbindung (Id')), mit einer Verbindung der Formel R2X in einem Lösungsmittel in Gegenwart einer Base bei einer Temperatur, die zwischen -30°C und +80°C liegt, reagieren lässt, wobei X ein Chlor-, Brom- oder Iodatom ist und R1 bis R4, B und n dieselbe Bedeutung wie in Anspruch 1 haben.

21. Verfahren nach Anspruch 20, **dadurch gekennzeichnet, dass** die Base ausgewählt ist aus der Gruppe, umfassend ein Alkoholat, ein metallisches Hydrid oder ein Amid, und das Lösungsmittel ausgewählt ist aus der Gruppe, umfassend die Ether, die cyclischen Ether, Dimethylformamid, Dimethylsulfoxid, die aromatischen Lösungsmittel.

22. Verfahren zur Herstellung von Verbindungen der Formel (I) (Verfahren D) nach Anspruch 1, in welchen W für die Gruppe S=O steht, **dadurch gekennzeichnet, dass** man eine S-Oxidation einer Verbindung der Formel (I), in welcher W ein Schwefelatom ist, bewirkt, wobei diese Oxidation von 2-Imidazolin-5-thionen mit einer stöchiometrischen Menge an Peroxiden in einem Lösungsmittel bei einer Temperatur, die zwischen -20°C und +20°C liegt, ausgeführt wird.

23. Verfahren nach Anspruch 22, **dadurch gekennzeichnet, dass** das Peroxid eine Persäure ist und das Lösungsmittel ausgewählt ist aus der Gruppe umfassend Chloroform und Methylenchlorid.

24. Verbindungen, welche insbesondere als Zwischenprodukte bei der Herstellung von Verbindungen nach einem der Ansprüche 1 bis 15 nützlich sind, **dadurch gekennzeichnet, dass** diese den folgenden Formeln IIa, III, V und VIIa: 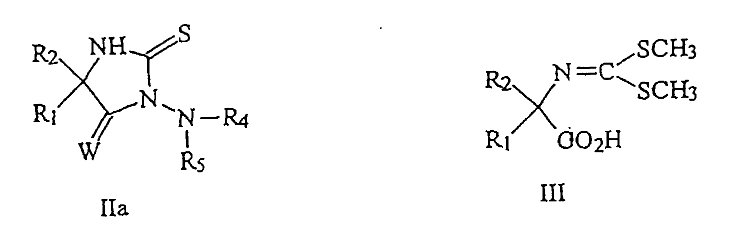 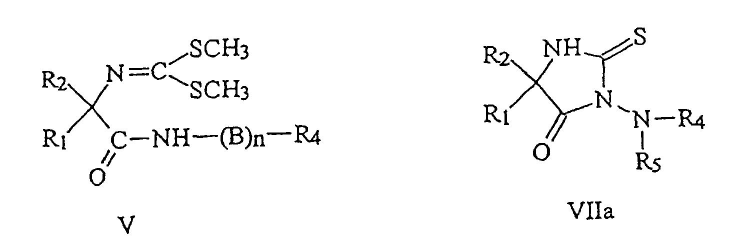 entsprechen, in welchen W, R1, R2, R4 und R5, n und B so wie in Anspruch 1 definiert sind, wobei R4 von einem Wasserstoffatom verschieden ist, ausschließlich der Verbindungen nach Formel IIa mit:
W = S
R1 = Methyl, R2 = Phenyl, R5 = Wasserstoff und R4 = Methyl oder Phenyl;
W = S
R1 = Ethyl, R2 = Phenyl, R4 = Methyl und R5 = Wasserstoff;
W = S
R1 = Methyl, R2 = p-Tolyl und
R4 = Methyl, R5 = Wasserstoff oder Methyl oder
R4 = Phenyl, R5 = Wasserstoff;
W = S,
R1 und R2 = Phenyl, R4 = Methyl und R5 = H,
und ausschließlich der Verbindungen nach Formel VIIa mit: R4 = Methyl, R5 = Methyl, R1 = Wasserstoff und R2 = Methyl oder Phenyl.

25. Verbindungen nach Anspruch 24, **dadurch gekennzeichnet, dass** R5 ein Wasserstoffatom ist,
R2 für eine Alkylgruppe mit 1 bis 3 Kohlenstoffatomen steht,
R1 für den Phenylring steht, der gegebenenfalls mit R7 substituiert ist,
R4 für den Phenylring steht, der gegebenenfalls mit R7 substituiert ist, und
R7 so wie in Anspruch 1 definiert ist.

26. Verbindungen nach Anspruch 24 oder 25, **dadurch gekennzeichnet, dass** diese der Formel VIIa entsprechen.

27. Verbindungen nach Anspruch 25 oder 26, **dadurch gekennzeichnet, dass** R2 für die Methylgruppe steht.

28. Verbindungen nach Anspruch 25, 26 oder 27, **dadurch gekennzeichnet, dass** R1 für den Phenylring steht.

29. Verfahren zur Herstellung von 2-Thiohydantoinen der Formel (VII) (Verfahren F): 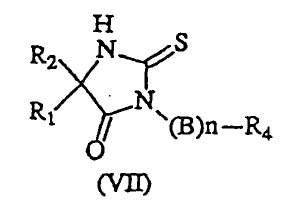 **dadurch gekennzeichnet, dass** man vor der Cyclisierung eine Verbindung der Formel (IV) R4(B)nNH2 mit Isocyanatderivaten von Aminosäuren der Formel (VIII): 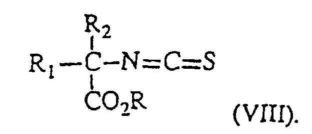 reagieren lässt, in welchen R1, R2, R4 dieselbe Bedeutung wie in Anspruch 1 haben, n = 1 ist und B für N(R5) steht.

30. Verfahren nach Anspruch 29, **dadurch gekennzeichnet, dass** die Cyclisierung thermisch durch Erwärmen der Mischung der Reaktanden bei einer Temperatur, die zwischen 110 und 180°C liegt, in einem aromatischen Lösungsmittel, wie z.B. Toluol, Xylol, Chlorbenzolen, durchgeführt wird.

31. Verfahren nach Anspruch 29, **dadurch gekennzeichnet, dass** die Cyclisierung mit einem Äquivalent an Base in einem Lösungsmittel und bei einer Temperatur, die zwischen -10 und +80°C liegt, durchgeführt wird, wobei das Milieu im folgenden bei Umgebungstemperatur neutralisiert wird.

32. Verfahren nach Anspruch 31, **dadurch gekennzeichnet, dass** die Base ausgewählt wird aus der Gruppe, umfassend ein Alkalialkoholat, ein Alkalihydroxid oder ein tertiäres Amin; wobei das Lösungsmittel ausgewählt wird aus der Gruppe, umfassend die Ether, die cyclischen Ether, die Alkohole, die Ester, DMF, DMSO.

33. Verfahren (Verfahren G) nach den Ansprüchen 29 und 31, **dadurch gekennzeichnet, dass** man, bevor man durch Neutralisation in saurem Milieu die 2-Thiohydantoinderivate erhält, ein Alkylhalogenid R3X einsetzt, um die Verbindung der Formel Id zu bilden, wobei X ein Chlor-, Brom- oder Iodatom oder eine Alkylsulfonyloxy- oder Arylsulfonyloxygruppe ist und R3 dieselbe Bedeutung wie in Anspruch 1 hat.

34. Fungizide Zusammensetzungen, welche in Verbindung mit einem oder mehreren festen oder flüssigen Trägern, welche landwirtschaftlich verträglich sind, und/oder grenzflächenaktiven Mitteln, die ebenfalls landwirtschaftlich verträglich sind, eine (oder mehrere) aktive Substanz(en) umfassen, die eine Verbindung der Formel I ist, wie sie in einem der Ansprüche 1 bis 15 definiert ist.

35. Fungizide Zusammensetzungen nach Anspruch 34, **dadurch gekennzeichnet, dass** diese 0,5 bis 95 Gew.-% der Verbindungen nach einem der Ansprüche 1 bis 15 enthalten.

36. Fungizide Zusammensetzungen nach Anspruch 34 oder 35, **dadurch gekennzeichnet, dass** diese Zusammenstellungen einer Verbindung der Formel I mit einer anderen aktiven Substanz enthalten.

37. Zusammensetzungen nach Anspruch 36, **dadurch gekennzeichnet, dass** die andere aktive Substanz ein Fungizid ist.

38. Zusammensetzungen nach Anspruch 36 oder 37, **dadurch gekennzeichnet, dass** man diese aus Granalien, welche eine Verbindung der Formel I umfassen, und aus Spritzpulver, aus Granalien oder aus wässrigen Suspensionen, welche eine andere aktive Substanz umfassen, erhalten kann.

39. Zusammensetzungen nach Anspruch 38, **dadurch gekennzeichnet, dass** die andere aktive Substanz ein Fungizid ist.

40. Verfahren zur Behandlung von Kulturen, welche an Pilzerkrankungen erkrankt sind oder für eine Erkrankung anfällig sind, **dadurch gekennzeichnet, dass** man zur Prävention oder Heilung eine wirksame Menge einer Verbindung nach einem der Ansprüche 1 bis 15 oder einer Zusammensetzung nach den Ansprüchen 34 bis 39 aufträgt.

41. Verfahren zur Behandlung nach Anspruch 40, **dadurch gekennzeichnet, dass** die wirksame Dosis zwischen 0,005 und 5 kg/ha liegt.

42. Verfahren zur Behandlung nach Anspruch 41, **dadurch gekennzeichnet, dass** die wirksame Dosis zwischen 0,01 und 1 kg/ha liegt.

## Claims

1. Compounds comprising an imidazolinone or imidazolinethione group, **characterized in that** they correspond to the general formula (I): 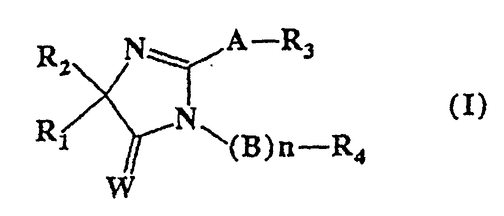 in which:
- W is the sulphur or oxygen atom or the S=O group,
- A represents O or S,
- n = 0 or 1,
- B represents N(R₅) or O or S or C(R₅)(R₆) or SO₂ or C=O,
- R₁ and R₂, which are identical or different, represent:
- H, provided that one of the 2 groups is other than H, or
- an alkyl or haloalkyl radical of 1 to 6 carbon atoms, or
- an alkoxyalkyl, alkylthioalkyl, alkylsulphonylalkyl, monoalkylaminoalkyl, alkenyl or alkynyl radical of 2 to 6 carbon atoms, or
- a dialkylaminoalkyl or cycloalkyl radical of 3 to 7 carbon atoms, or
- an aryl radical, comprising phenyl, naphthyl, thienyl, furyl, pyridyl, benzothienyl, benzofuryl, quinolinyl, isoquinolinyl or methylenedioxyphenyl, optionally substituted by 1 to 3 groups chosen from R₇, or
- an arylalkyl, aryloxyalkyl, arylthioalkyl or arylsulphonylalkyl radical, the terms aryl and alkyl having the definitions given above, or
- R₁ and R₂ can form, with the carbon to which they are bonded on the ring, a carbocycle or heterocycle having from 5 to 7 atoms, it being possible for these rings to be fused with a phenyl, optionally substituted by 1 to 3 groups chosen from R₇;
- R₃ represents:
- an alkyl group of 1 to 6 carbon atoms, or
- an alkoxyalkyl, alkylthioalkyl, alkylsulphonylalkyl, haloalkyl, cyanoalkyl, thiocyanatoalkyl, oxoalkyl, alkenyl or alkynyl group of 2 to 6 carbon atoms, or
- a dialkylaminoalkyl, alkoxycarbonylalkyl or N-alkylcarbamoylalkyl group of 3 to 6 carbon atoms, or
- an N,N-dialkylcarbamoylalkyl group of 4 to 8 carbon atoms, or
- an arylalkyl group, the alkyl part being a radical of 1 to 6 carbon atoms and the aryl part being phenyl, naphthyl, thienyl, furyl or pyridyl, optionally substituted by 1 to 3 groups chosen from R₇;
- R₄ represents:
- the hydrogen atom when n is equal to 1, or
- an alkyl group of 1 to 6 carbon atoms, or
- an alkoxyalkyl, alkylthioalkyl, haloalkyl, cyanoalkyl, thiocyanatoalkyl, alkenyl or alkynyl group of 2 to 6 carbon atoms, or
- a dialkylaminoalkyl, alkoxycarbonylalkyl or N-alkylcarbamoylalkyl group of 3 to 6 carbon atoms, or
- an N,N-dialkylcarbamoylalkyl group of 4 to 8 carbon atoms, or
- an aryl radical, comprising phenyl, naphthyl, thienyl, furyl, pyridyl, pyrimidyl, pyridazinyl, pyrazinyl, benzothienyl, benzofuryl, quinolinyl, isoquinolinyl or methylenedioxyphenyl, optionally substituted by 1 to 3 groups chosen from R₇, or
- an arylalkyl, aryloxyalkyl, arylthioalkyl or arylsulphonylalkyl radical, the terms aryl and alkyl having the definitions given above, or
- an amino group disubstituted by 2 identical or different groups chosen from:
- an alkyl radical of 1 to 6 carbon atoms,
- an alkoxyalkyl, alkenyl or alkynyl radical of 3 to 6 carbon atoms,
- a cycloalkyl radical of 3 to 7 carbon atoms,
- an arylalkyl, as defined above, phenyl or naphthyl radical, optionally substituted by 1 to 3 groups chosen from R₇, or
- a thienylmethyl or furfuryl radical,
- a pyrrolidino, piperidino, morpholino or piperazino group optionally substituted by an alkyl radical of 1 to 3 carbon atoms;
- R₅ represents:
- H, except when R₄ is H, or
- an alkyl, haloalkyl, alkylsulphonyl or haloalkylsulphonyl radical of 1 to 6 carbon atoms, or
- an alkoxyalkyl, alkylthioalkyl, acyl, alkenyl, alkynyl, haloacyl, alkoxycarbonyl, haloalkoxycarbonyl, alkoxyalkylsulphonyl or cyanoalkylsulphonyl radical of 2 to 6 carbon atoms, or
- an alkoxyalkoxycarbonyl, alkylthioalkoxycarbonyl or cyanoalkoxycarbonyl radical of 3 to 6 carbon atoms, or
- the formyl radical, or
- a cycloalkyl, alkoxyacyl, alkylthioacyl, cyanoacyl, alkenylcarbonyl or alkynylcarbonyl radical of 3 to 6 carbon atoms, or
- a cycloalkylcarbonyl radical of 4 to 8 carbon atoms, or
- a phenyl radical; an arylalkylcarbonyl radical, in particular a phenylacetyl or phenylpropionyl radical; an arylcarbonyl radical, in particular a benzoyl radical, optionally substituted by 1 to 3 groups chosen from R₇; a thienylcarbonyl radical; a furyl-carbonyl radical; a pyridylcarbonyl radical; a benzyloxycarbonyl radical; a furfuryloxycarbonyl radical; a tetrahydrofurfuryloxycarbonyl radical; a thienylmethoxycarbonyl radical; a pyridylmethoxycarbonyl radical; a phenoxycarbonyl or phenylthiocarbonyl radical, the phenyl being itself optionally substituted by 1 to 3 groups chosen from R₇; an alkylthiocarbonyl radical; a haloalkylthiocarbonyl radical; an alkoxyalkylthiocarbonyl radical; a cyanoalkylthiocarbonyl radical; a benzylthiocarbonyl radical; a furfurylthiocarbonyl radical; a tetrahydrofurfurylthiocarbonyl radical; a thienylmethylthiocarbonyl radical; a pyridylmethylthiocarbonyl radical; or an arylsulphonyl radical, or
- a carbamoyl radical, optionally mono- or disubstituted by
- an alkyl or haloalkyl group of 1 to 6 carbon atoms, or
- a cycloalkyl, cycloalkenyl or cycloalkynyl group of 3 to 6 carbon atoms, or
- an alkoxyalkyl, alkylthioalkyl or cyanoalkyl group of 2 to 6 carbon atoms, or
- a phenyl, optionally substituted by 1 to 3 R₇ groups;
- a sulphamoyl group, optionally mono- or disubstituted by
- an alkyl or haloalkyl group of 1 to 6 carbon atoms, or
- a cycloalkyl, cycloalkenyl or cycloalkynyl group of 3 to 6 carbon atoms, or
- an alkoxyalkyl, alkylthioalkyl or cyanoalkyl group of 2 to 6 carbon atoms, or
- a phenyl, optionally substituted by 1 to 3 R₇ groups;
- an alkylthioalkylsulphonyl group of 3 to 8 carbon atoms or a cycloalkylsulphonyl group of 3 to 7 carbon atoms;
- R₆ represents:
- the hydrogen atom, or
- the cyano group, or
- an alkyl group of 1 to 6 carbon atoms or a cycloalkyl group of 3 to 7 carbon atoms, or
- an acyl or alkoxycarbonyl group of 2 to 6 carbon atoms, or
- the benzoyl group, optionally substituted by 1 to 3 R₇ groups;
- R₇ represents:
- a halogen atom, or
- an alkyl, haloalkyl, alkoxy, haloalkoxy, alkylthio, haloalkylthio or alkylsulphonyl radical of 1 to 6 carbon atoms, or
- a cycloalkyl, halocycloalkyl, alkenyloxy, alkynyloxy, alkenylthio or alkynylthio radical of 3 to 6 carbon atoms, or
- the nitro or cyano group, or
- an amino radical, optionally mono- or disubstituted by an alkyl or acyl radical of 1 to 6 carbon atoms or an alkoxycarbonyl radical of 2 to 6 carbon atoms, or
- a phenyl, phenoxy or pyridyloxy radical, these radicals optionally being substituted;
and their salified forms,
with the exclusion of the compounds for which, when R₁ and R₂ are simultaneously phenyl or simultaneously methyl, B is C(R₅)(R₆), A is sulphur and W is O or S;
with the exclusion of the 3 compounds with R₁ = hydrogen, n = 1, B = NR₅, with R₅ and R₄ = methyl, AR₃ = SCH₃, W = O and R₂ being either a hydrogen, a methyl or a phenyl;
with the exclusion of the compound in which R₁ is a phenyl, R₂, R₃ and R₄ are methyl, n is equal to zero, and A and W are each the sulphur atom;
with the exclusion also of the compounds of formula (I) for which:
- n = 0; and
- R₁ and R₂ simultaneously represent a methyl; and
- A is a sulphur atom; and
- R₄ is a phenyl substituted by 2 groups:
- one chosen from the cyano or nitro groups or a halogen atom;
- the other chosen from a trifluoromethyl group or a halogen atom;
and with the exclusion of the following compounds:
- 3-[(4-chlorophenyl)sulphonyl]-3,5-dihydro-2-methoxy-5-methyl-5-(1-methylethyl)-4H-imidazol-4-one;
- 2-(ethoxycarbonylmethylthio)-3-acetyl-5,5-diphenyl-3,5-dihydro-4H-imidazol-4-one
- 2-butoxy-5-ethyl-3,5-dihydro-3-methyl-5-phenyl-4H-imidazol-4-one;
- 2-methoxy-5-methyl-3,5-dihydro-3-(1-phenylethyl)-4H-imidazol-4-one;
- 2-methylthio-5,5-dimethyl-3,5-dihydro-3-phenyl-4H-imidazol-4-one;
- 2-(methylthio)-3,5,5-triphenyl-3,5-dihydro-4H-imidazol-4-one;
- 3-methyl-2-(methylthio)-1,3-diazaspiro[4.5]dec-1-en-4-one;
- 3-methyl-2-(methylthio)-1,3-diazaspiro[4.5]dec-1-ene-4-thione.

2. Compounds according to Claim 1, **characterized in that** A is the sulphur atom.

3. Compounds according to Claim 1, **characterized in that** W is the oxygen atom.

4. Compounds according to Claim 2, **characterized in that** they correspond to the general formula: 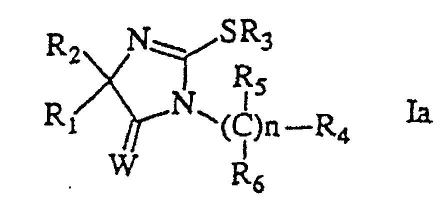 in which W, n and R₁ to R₆ have the same meaning as in Claim 1.

5. Compounds according to Claim 2, **characterized in that** they correspond to the general formula: 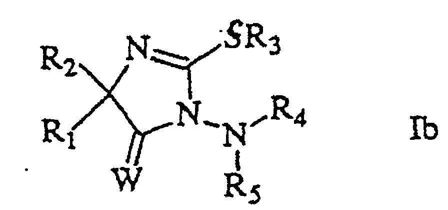 in which W and R₁ to R₅ have the same meaning as in Claim 1.

6. Compounds according to Claim 2, **characterized in that** they correspond to the general formula: 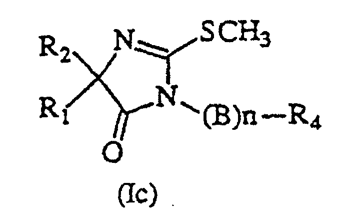 in which B, n and R₁ to R₄ have the same meaning as in Claim 1.

7. Compounds according to Claim 2, **characterized in that** they correspond to the general formula: 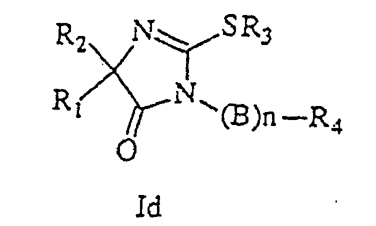 in which B, n and R₁ to R₄ have the same meaning as in Claim 1.

8. Compounds according to one of Claims 1 to 7, **characterized in that** R₅ is the hydrogen atom.

9. Compounds according to Claim 5, **characterized in that** R₅ is the hydrogen atom (compound Ib').

10. Compounds according to one of Claims 1 to 9, **characterized in that** R₁ and R₂ are other than the hydrogen atom.

11. Compounds according to one of Claims 1 to 10, **characterized in that** R₂ is an alkyl group of 1 to 3 carbon atoms.

12. Compounds according to one of Claims 1 to 11, **characterized in that** R₁ is a phenyl, optionally substituted by an R₇ group.

13. Compounds according to one of Claims 1 to 12, **characterized in that** R₃ is an alkyl group of 1 to 3 carbon atoms.

14. Compounds according to one of Claims 1 to 13, **characterized in that** R₄ is a phenyl, optionally substituted by an R₇ group.

15. Compounds according to one of Claims 1 to 14, **characterized in that** R₃ is a methyl radical.

16. Process for the preparation (process A) of the compounds of formula (I) according to Claim 2, **characterized in that** 2-thiohydantoins of formula (II) are S-alkylated with a compound of formula R₃X, in the presence of a base and solvent, according to the scheme: 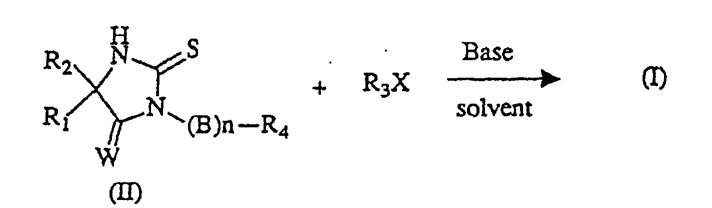 in which R₁, R₂, R₃, R₄, B and W have the meaning of Claim 1 and X is the chlorine, bromine or iodine atom or the sulphate group or an alkylsulphonyloxy or arylsulphonyloxy group.

17. Process for the preparation of the 2-methylthio-2-imidazolin-5-one compounds of formula (Ic) (process B) according to Claim 6, **characterized in that** a compound of formula (V) is cyclized, by simple heating in an aromatic solvent at reflux, according to the scheme: 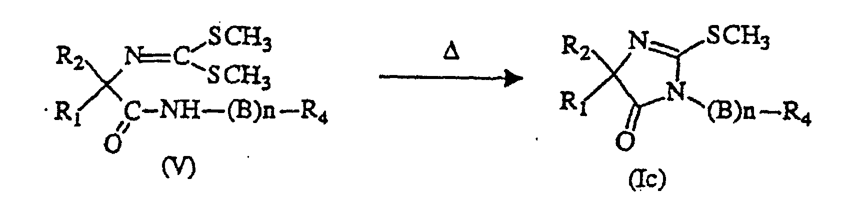 in which R₁, R₂, R₄, B and n have the same meaning as in Claim 1.

18. Process for the preparation of the compounds of formula (V) (process B) according to Claim 17, **characterized in that** an iminodithiocarbonate of formula (III) is condensed with a hydrazine of formula (IV) according to the scheme: 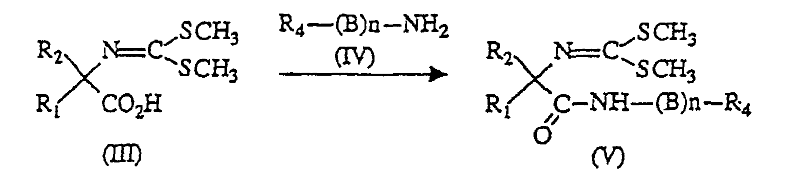 in which:
- R₁, R₂ and R₄ have the same meaning as in Claim 1,
- n is equal to 1 and B represents NR₅,
- R₅ has the same meaning as in Claim 1;
the acid (III) having to be activated for this condensation in the acid chloride form, in the dicyclohexylisourea form, using dicyclohexylcarbodiimide, or in the imidazolide form, using carbonyldiimidazole.

19. Process for the preparation of the compounds of formula (Ib) (process C1), according to the scheme: 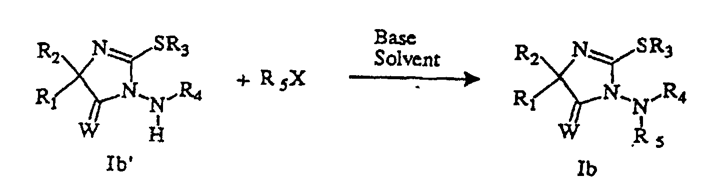 **characterized in that** a compound of formula R₅X is reacted, in a solvent in the presence of a base, with a compound of formula (Ib) according to Claim 5, in which the R₅ group is the hydrogen atom according to Claim 9 (compound (Ib')), the R₁ to R₄ groups have the meaning given in Claim 1, the R₅ group is an alkyl, alkoxycarbonyl, acyl, aroyl, alkylsulphonyl, arylsulphonyl, carbamoyl or sulphamoyl group and X represents a leaving group, such as a halogen, the sulphate group, an optionally substituted phenoxy, R₅O when R₅ is acyl, or an alkylsulphonyloxy or arylsulphonyloxy group, or a nonleaving group, such as isocyanate or isothiocyanate.

20. Process for the preparation of the compounds of formula (Id) (process C2), according to the scheme: 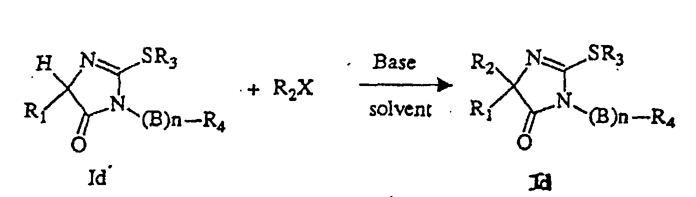 **characterized in that** a compound of formula (Id) according to Claim 7, in which R₂ is the hydrogen atom (compound Id')), is reacted with a compound of formula R₂X, in a solvent and in the presence of a base, at a temperature of between -30°C and +80°C, X being a chlorine, bromine or iodine atom and R₁ to R₄, B and n having the same meaning as in Claim 1.

21. Process according to Claim 20, **characterized in that** the base is chosen from the group consisting of an alkoxide, a metal hydride and an amide and the solvent is chosen from the group consisting of ethers, cycloethers, dimethylformamide, dimethyl sulphoxide and aromatic solvents.

22. Process for the preparation of the compounds of formula (I) (process D) according to Claim 1, in which compounds W represents the S=O group, **characterized in that** an S-oxidation of a compound of formula (I) in which W is the sulphur atom is carried out, this oxidation of 2-imidazoline-5-thiones being carried out with a stoichiometric amount of peroxides in a solvent at a temperature of between -20°C and +20°C.

23. Process according to Claim 22, **characterized in that** the peroxide is a peracid and the solvent is chosen from the group consisting of chloroform and methylene chloride.

24. Compounds of use in particular as intermediates in the preparation of the compounds according to one of Claims 1 to 15, **characterized in that** they correspond to the formulae IIa, III, V and VIIa: 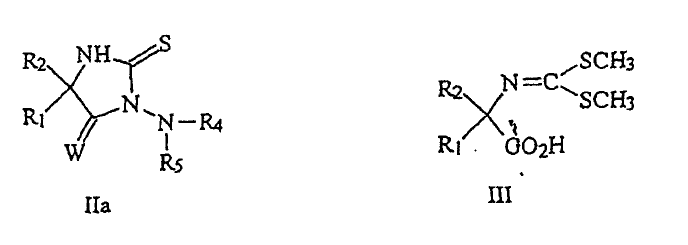 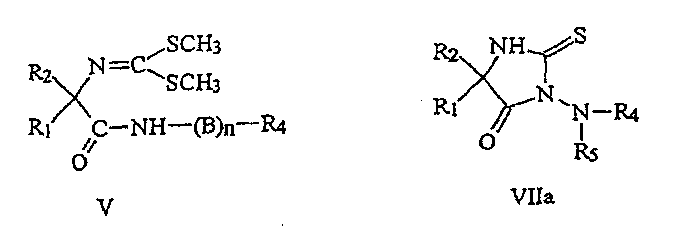 in which W, R₁, R₂, R₄, R₅, n and B are as defined in Claim 1, R₄ being other than the hydrogen atom, with the exclusion of the compounds according to formula IIa with:
W = S,
R₁ = methyl, R₂ = phenyl, R₅ = hydrogen and R₄ = methyl or phenyl;
W = S,
R₁ = ethyl, R₂ = phenyl, R₄ = methyl and R₅ = hydrogen;
W = S,
R₁ = methyl, R₂ = p-tolyl, and
R₄ = methyl, R₅ = hydrogen or methyl, or
R₄ = phenyl, R₅ = hydrogen;
W = S,
R₁ and R₂ = phenyl, R₄ = methyl and R₅ = H,
and with the exclusion of the compounds according to the formula VIIa with:
R₄ = methyl, R₅ = methyl, R₁ = hydrogen and
R₂ = methyl or phenyl.

25. Compounds according to Claim 24, **characterized in that**
R₅ is the hydrogen atom,
R₂ represents an alkyl group of 1 to 3 carbon atoms,
R₁ represents the phenyl nucleus, optionally substituted by R₇,
R₄ represents the phenyl nucleus, optionally substituted by R₇, and
R₇ is as defined in Claim 1.

26. Compounds according to Claim 24 or 25, **characterized in that** they correspond to the formula VIIa.

27. Compounds according to Claim 25 or 26, **characterized in that** R₂ represents the methyl group.

28. Compounds according to Claim 25, 26 or 27, **characterized in that** R₁ represents the phenyl nucleus.

29. Process for the preparation of the 2-thiohydantoins of formula (VII) (process F): 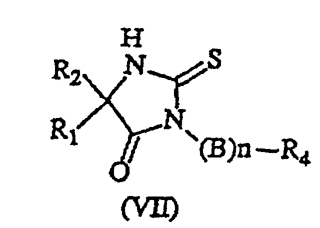 **characterized in that**, before cyclization, a compound of formula (IV) R₄(B)ₙNH₂ is reacted with isothiocyanates derived from amino acids of formula (VIII): 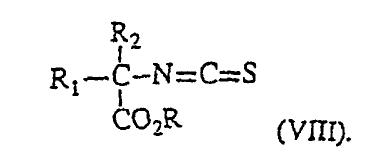 in which hydantoins R₁, R₂ and R₄ have the same meaning as in Claim 1, n = 1 and B represents N(R₅).

30. Process according to Claim 29, **characterized in that** the cyclization is carried out thermally by heating the mixture of the reactants at a temperature of between 110 and 180°C in an aromatic solvent, such as toluene, xylene or chlorobenzenes.

31. Process according to Claim 29, **characterized in that** the cyclization is carried out with one equivalent of base in a solvent and at a temperature of between -10 and +80°C, the mixture subsequently being neutralized at ambient temperature.

32. Process according to Claim 31, **characterized in that** the base is chosen from the group consisting of an alkaline alkoxide, an alkaline hydroxide or a tertiary amine; the solvent is chosen from the group consisting of ethers, cyclic ethers, alcohols, esters, DMF and DMSO.

33. Process (process G) according to Claims 29 and 31, **characterized in that**, before arriving at the 2-thiohydantoin derivatives by neutralization in an acidic medium, an alkyl halide R₃X is charged in order to form the compound of formula Id, X being a chlorine, bromine or iodine atom or an alkylsulphonyloxy or arylsulphonyloxy group and R₃ having the same meaning as in Claim 1.

34. Fungicidal compositions comprising, in combination with one or more solid or liquid vehicles acceptable in agriculture and/or surface-active agents, also acceptable in agriculture, one (or more than one) active material which is a compound of formula I as defined in one of Claims 1 to 15.

35. Fungicidal compositions according to Claim 34, **characterized in that** they comprise from 0.5% to 95% by weight of compounds according to one of Claims 1 to 15.

36. Fungicidal compositions according to Claim 34 or 35, **characterized in that** they comprise combinations of a compound of formula I with another active material.

37. Compositions according to Claim 36, **characterized in that** the other active material is a fungicide.

38. Compositions according to Claim 36 or 37, **characterized in that** they can be obtained from granules which comprise a compound of formula I and from wettable powders, granules or aqueous suspensions which comprise the other active material.

39. Compositions according to Claim 38, **characterized in that** the other active material is a fungicide.

40. Process for the treatment of crops attacked by or liable to be attacked by fungal diseases, **characterized in that** an effective amount of a compound according to one of Claims 1 to 15 or of a composition according to Claims 34 to 39 is applied, preventively or curatively.

41. Treatment process according to Claim 40, **characterized in that** the effective dose is between 0.005 and 5 kg/ha.

42. Treatment process according to Claim 41, **characterized in that** the effective dose is between 0.01 and 1 kg/ha.
